# EUROPEAN PATENT SPECIFICATION

(11) **EP 2 538 928 B1**
(45) Date of publication and mention of the grant of the patent: **03.05.2017**
(21) Application number: 11705797.6
(22) Date of filing: 23.02.2011
(51) Int. Cl.: A61K 9/26, A61K 31/485

(54) **ABUSE-RESISTANT FORMULATIONS**
MISSBRAUCHSRESISTENTE FORMULIERUNGEN
FORMULATIONS À L'ÉPREUVE D'UN USAGE ABUSIF

(30) Priority: 24.02.2010 US 307588 P
(43) Date of publication of application: 02.01.2013
(73) Proprietor: Cima Labs Inc., Brooklyn Park, Minnesota 55428 (US)
(72) Inventor: HAMED, Ehab, Maple Grove Minnesota 55311 (US); KRALING, Carrie, Minneapolis Minnesota 55405 (US)
(74) Representative: Hallybone, Huw George
(86) International application number: PCT/US2011/025914
(87) International publication number: WO 2011/106416

(56) References cited:
- WO-A1-2009/035474
- US-A- 4 959 219
- US-A1- 2007 141 147

## Description

### TECHNICAL FIELD

This invention relates to a sustained-release oral dosage form of hydrocodone suitable for twice daily dosing.

### BACKGROUND

Hydrocodone is administered to patients to reduce pain. Successful pain management in many of these patients requires maintenance of certain blood levels of hydrocodone throughout the day. One way of obtaining acceptable blood levels, used commonly in the pharmaceutical industry, is providing a dose which contains far more drug than is necessary to obtain the desired blood level. Blood levels shortly after the tablet is ingested reach a maximum or Cₘₐₓ in a relatively short time, often within hours of ingestion (Tₘₐₓ) and thereafter, as the body uses, processes and excretes drug from the blood system, the blood level drops. If the Cₘₐₓ attained is sufficiently high, and the body's clearance of the drug is sufficiently slow, the blood levels may not fall to sub-therapeutic levels for 4-12 hours or even longer. With drugs like hydrocodone, however, this is an impractical and inefficient dosing system. In addition, there is a risk to the patient in that such high initial API levels can cause significant side effects.

Another method of administering hydrocodone involves the use of an extended release mechanism. An extended release can be achieved in many different ways and there are many different release profiles that can be attained. Not only could this strategy reduce the number of doses that need to be taken in a day, it also may prevent one from being exposed to the side effects which can come from unnecessarily high initial blood levels.

Those who seek to abuse hydrocodone to "get high" can be frustrated by such extended and indeed other controlled release strategies. These strategies actively prevent one from obtaining high blood levels of the drug which can cause the euphoria or other physiologic effects which they are actually seeking, but which normal patients would consider an undesirable or even dangerous side effect. Such prescription drug abusers have learned to circumvent controlled release mechanisms by various administrative abuse means including simply chewing extended release tablets or crushing them using a mortar and a pestle for injection or the like. Another way to circumvent controlled release coatings is to attempt to dissolve the dosage form in a solvent such as water or ethanol. The latter can be particularly dangerous as hydrocodone should not be taken with alcohol. Depending upon the extended release formulation, the ethanol or water may act as a solvent, dissolving or eroding the dosage form and circumventing the intended controlled release. The resulting material can then be administered generally, orally, or in a syringe by a drug abuser.

Such abuse can have rather far ranging consequences. For example, cancer patients, patients with post-operative or pre-operative pain, and patients with chronic pains from arthritis or back injuries need to have useful drugs (e.g., hydrocodone) available to them. The potential for abuse, however, is a constant concern to regulators and law enforcement as these prescription drugs may be more freely obtainable than truly illegal illicit substances. There are also the societal problems relating to drug use, which includes the cost of their health care, the cost of their rehabilitation, the increase in crime which may come from supporting their drug habit, and the like.

US 4,959,219 discloses coatings for individual ion exchange resin particles comprised of ethylcellulose and corn oil or acetylated monoglycerides.

### SUMMARY

The invention is defined by the appended claims.

Sustained-release oral dosage forms suitable for twice-a-day administration of hydrocodone are provided. The dosage form includes a matrix having a first viscosity modifier and coated granules comprising hydrocodone or a salt form thereof (e.g., hydrocodone bitartrate). In some cases, a dosage form, as described herein, has a release profile such that after 6 hours in 500 ml of 0.1N hydrochloric acid, less than about 80 percent of the hydrocodone is released. In addition, a dosage form may have alcohol resistance, crush resistance and/or resistance to food effect. Dosage forms that are resistant to food effect are further described below. Formulations that are resistant to food effect can also be described as having Tₘₐₓ changes of less than 2, 1.5, or 1 hour when the fed measured Tₘₐₓ is compared to the fasted measured Tₘₐₓ. One of ordinary skill in the art will appreciate that formulations that are alcohol resistant, crush resistant and/or resistant to food effect are generally safer, because their safety is not as reliant upon patient compliance.

Provided herein is a sustained-release oral dosage form suitable for twice-a-day administration comprising: a matrix, wherein the matrix comprises a first viscosity modifier in an amount from 1 to 10 percent by weight of the dosage form; and coated granules comprising (i) a granule, the granule comprising hydrocodone or a salt form thereof, such as hydrocodone bitartrate, in an amount from 0.1 to 90 percent by weight of the granule, a first strong film former in an amount from 1 to 90 percent by weight of the granule, a second viscosity modifier in an amount from 1 to 90 percent by weight of the granule, wherein the granule does not contain a fat/wax, and (ii) a coating on the granule, wherein the coating is present in an amount from 20 to 80 percent by weight of the coated granule, wherein the coating comprises a second strong film former in an amount from 10 to 50 percent by weight of the coated granule, and a fat/wax in an amount from 10 to 25 percent by weight of the coated granule. In some embodiments, the release of hydrocodone from the dosage form after 6 hours is less than about 80 percent. In some embodiments, the release of the hydrocodone from the dosage form after 10 hours is less than about 85 percent.

In some embodiments, the percent of hydrocodone released after 2 hours in a solution of 0.1N hydrochloric acid and 40% alcohol is no more than 10 percentage points greater than the percent of hydrocodone released in a solution of 0.1N hydrochloric acid in the absence of alcohol. In some embodiments, the release of hydrocodone from the dosage form 30 minutes after simulated oral tampering is less than about 50 percent.

The first viscosity modifier is selected from the group consisting of: sodium alginate, hydroxypropylmethylcellulose, hydroxyethylcellulose, hydroxypropylcellulose, methylcellulose, carboxymethylcellulose, sodium carboxymethylcellulose, polyvinylpyrrolidone, crosslinked polyacrylic acid, gelatin, pectins, gums, polyethylene oxides, Konjac flour, carrageenan, xanthan gum, or mixtures thereof. For example, the first viscosity modifier can be a gelling polymer. In some embodiments, the gelling polymer is selected from the group consisting of:
hydroxypropylmethylcellulose, hydroxypropylcellulose, methylcellulose, hydroxyethylcellulose, and carboxymethylcellulose. For example, in some cases a gelling polymer can be hydroxypropylmethylcellulose.

In some embodiments, the first viscosity modifier is present in an amount from about 5 to about 10 percent by weight of the dosage form. In some embodiments, the first viscosity modifier is present in an amount from about 6 percent by weight of the dosage form. In some embodiments, the first viscosity modifier is present in an amount from about 10 percent by weight of the dosage form.

A coated granule, as described herein, comprises: a granule comprising hydrocodone or a salt form thereof in an amount from 0.1 to 90 percent by weight of the granule, a first strong film former in an amount from 1 to 90 percent by weight of the granule, a second viscosity modifier in an amount from 1 to 90 percent by weight of the granule, wherein the granule does not contain a fat/wax; and a coating on the granule, wherein the coating is present in an amount from 20 to 80 percent by weight of the coated granule, and wherein the coating comprises a second strong film former in an amount from 10 to 50 percent by weight of the coated granule, and a fat/wax in an amount from 10 to 25 percent by weight of the coated granule.

The first and second strong film formers are independently selected from the group consisting of: ethylcellulose; Ammonio Methacrylate Copolymer, Type B; Ammonio Methacrylate Copolymer, Type A; Amino Methacrylate Copolymer; Ethyl Acrylate and Methyl Methacrylate Copolymer Dispersion; Methacrylic Acid Copolymer, Type A; Methacrylic Acid Copolymer, Type B; and shellac. In some embodiments, the first and second strong film formers are ethylcellulose. In some embodiments, the first strong film former and the second strong film former are the same.

In some embodiments, the first strong film former is present in an amount from about 30 to about 80 percent by weight of the granule. For example, the first strong film former can be present in an amount from about 40 to about 70 percent by weight of the granule.

The second viscosity modifier is selected from the same group as defined above for the first viscosity modifier. The second viscosity modifier is selected from the group consisting of: sodium alginate, hydroxypropylmethylcellulose, hydroxyethylcellulose, hydroxypropylcellulose, methylcellulose, carboxymethylcellulose, sodium carboxymethylcellulose, polyvinylpyrrolidone, crosslinked polyacrylic acid, gelatin, pectins, gums, polyethylene oxides, Konjac flour, carrageenan, xanthan gum, or mixtures thereof. In some embodiments, the second viscosity modifier is selected from the group consisting of: hydroxypropylmethylcellulose, hydroxypropylcellulose, methylcellulose, hydroxyethylcellulose, and carboxymethylcellulose. For example, the second viscosity modifier can be hydroxypropylmethylcellulose.

In some embodiments, the second viscosity modifier is present in an amount from about 10 to about 70 percent by weight of the granule. For example, the second viscosity modifier can be present in an amount from about 15 to about 40 percent by weight of the granule.

The fat/wax can be selected from the group consisting of: glycerol fatty esters, fatty glyceride derivatives, waxes, or fatty alcohols. For example, the fat/wax can be selected from the group consisting of: glyceryl behenate, glycerol palmitostearate, stearoyl macroglycerides, carnauba wax, bees wax, microcrystalline wax, and cetyl alcohol. In some embodiments, the fat/wax is glyceryl behenate.The fat/wax is present in an amount from 10 to 25 percent by weight of the coated granule. The granule does not contain a fat/wax and the coating on the granule contains a fat/wax in an amount from 10 to 25 percent by weight of the coated granule.

In some embodiments, the hydrocodone salt is hydrocodone bitartrate. In some embodiments, the hydrocodone or salt form thereof is present in an amount from about 1 to about 60 percent by weight of the granule. For example, the hydrocodone or salt form thereof is present in an amount from about 5 to about 35 percent by weight of the granule.

The granules are coated and in some embodiments, the coating is present in an amount from about 30 to about 70 percent by weight of the coated granule. For example, the coating can be present in an amount from about 30 to about 55 percent by weight of the coated granule.

In some embodiments, the coated granule comprises less than about 10 percent water per weight of the coated granule. For example, the coated granule comprises less than about 6 percent water per weight of the coated granule.

The sustained-release oral dosage form suitable for twice-a-day administration comprises: a matrix, wherein the matrix comprises a first viscosity modifier in an amount from 1 to 10 percent by weight of the dosage form; and coated granules, wherein the coated granules comprise: a granule comprising hydrocodone or a salt form thereof in an amount from 0.1 to 90 percent by weight of the granule, a first strong film former in an amount from 1 to 90 percent by weight of the granule, a second viscosity modifier in an amount from 1 to 90 percent by weight of the granule, wherein the granule does not contain a fat/wax; and a coating on the granule, wherein the coating is present in an amount from 20 to 80 percent by weight of the coated granule, and wherein the coating comprises a second strong film former in an amount from 10 to 50 percent by weight of the coated granule, and a fat/wax in an amount from 10 to 25 percent by weight of the coated granule.

In some cases, the dosage form can comprise a matrix, wherein the matrix comprises a first viscosity modifier in an amount from 1 to 10, 20 or 30 percent by weight of the dosage form; and coated granules, wherein the coated granules comprise: a granule comprising hydrocodone or a salt form thereof in an amount from about 1 to about 60 percent by weight of the granule, a first strong film former in an amount from about 30 to about 80 percent by weight of the granule, and a second viscosity modifier in an amount from about 10 to about 70 percent by weight of the granule, and a coating on the granule, wherein the coating is present in an amount from about 30 to about 70 percent by weight of the coated granule, and wherein the coating comprises a second strong film former in an amount from 10 to 50 percent by weight of the coated granule, and a fat/wax in an amount from 10 to 25 percent by weight of the coated granule.

In some cases, the dosage form can comprise a matrix, wherein the matrix comprises hydroxypropylmethylcellulose in an amount from about 1 to about 10 percent by weight of the dosage form; and coated granules, wherein the coated granules comprise: a granule comprising hydrocodone in an amount from about 5 to about 35 percent by weight of the granule, ethylcellulose in an amount from about 40 to about 70 percent by weight of the granule, hydroxypropylmethylcellulose in an amount from about 15 to about 40 percent by weight of the granule; and a coating on the granule, wherein the coating is present in an amount from about 30 to about 55 percent by weight of the coated granule, and wherein the coating comprises ethylcellulose in an amount from about 10 to about 50 percent by weight of the coated granule, and glyceryl behenate in an amount from about 10 to about 25 percent by weight of the coated granule.

The dosage form may be resistant to food effect. Resistance to food effect is measured using the methodology described in Example 4, provided herein. Generally, resistance to food effect is identified by comparing pharmacokinetic parameters from subjects that are fasted to those that are fed, e.g., have consumed a standard diet prior to administration. In some situations a standard diet can be high fat (i.e., about 50% of the calories are from fat), high carbohydrate or any other standard diet. A dosage form that is
resistant to food effect will show a smaller percent change (the difference between the fed and fasted pharmacokinetic parameter divided by the fasted pharmacokinetic parameter) in a given pharmacokinetic parameter compared to another formulation that is less resistant to food effect. Pharmacokinetic parameters that are useful for comparison include Cmax, and Tmax. One or more of these pharmacokinetic parameters can be compared at various time points. For example, the formulation described and tested in Example 4, below, showed a percent change of Tmax of 25%. That change in Tmax can be compared to Example 5. The data in Example 5 showed a percent change in Tmax of 38%. Therefore, the formulation in Example 5 was not as resistant to food effect as the formulation in Example 4. Notably the matrix in Example 5 comprised fat/wax. In some examples the food effect resistant formulations will have a percent change in Tmax of less than 35%, 30%, 25%, 20%, 15%, 10%, or 5%. Food effect resistant formulations can also provide percent changes in Cmax of less than 60%, 55%, 50%, 45%, 40%, 35%, 30%, 25%, 20%, 15%, 10% or 5%.

Further provided herein is a dosage form comprising: a matrix, wherein the matrix comprises hydroxypropylmethylcellulose in an amount of about 1 to about 10 percent by weight of the dosage form; and coated granules, wherein the coated granules comprise: a granule comprising hydrocodone in an amount of about 27 percent by weight of the granule, ethylcellulose in an amount from about 40 to about 70 percent by weight of the granule, and hydroxypropylmethylcellulose in an amount from about 15 to about 40 percent by weight of the granule; and a coating on the granule, wherein the coating is present in an amount from about 30 to about 55 percent by weight of the coated granule, and wherein the coating consists essentially of ethylcellulose in an amount from about 10 to about 50 percent by weight of the coated granule, and glyceryl behenate in an amount from about 10 to about 25 percent by weight of the coated granule.

Further provided herein is a dosage form comprising: a matrix, wherein the matrix comprises hydroxypropylmethylcellulose in an amount of about 1 to about 10 percent by weight of the dosage form; and coated granules, wherein the coated granules comprise: a granule comprising hydrocodone in an amount of about 9 percent by weight of the granule, ethylcellulose in an amount from about 40 to about 70 percent by weight of the granule, and hydroxypropylmethylcellulose in an amount from about 15 to about 40 percent by weight of the granule; and a coating on the granule, wherein the coating is present in an amount from about 30 to about 55 percent by weight of the coated granule, and wherein the coating consists essentially of ethylcellulose in an amount from about 10 to about 50 percent by weight of the coated granule, and glyceryl behenate in an amount from about 10 to about 25 percent by weight of the coated granule.

Further provided herein is a dosage form comprising: a matrix, wherein the matrix comprises hydroxypropylmethylcellulose in an amount of about 5 to about 10 percent by weight of the dosage form; and coated granules, wherein the coated granules comprise: a granule comprising hydrocodone in an amount of about 5 to about 35 percent by weight of the granule, ethylcellulose in an amount from about 40 to about 70 percent by weight of the granule, and hydroxypropylmethylcellulose in an amount of about 30 percent by weight of the granule; and a coating on the granule, wherein the coating is present in an amount from about 30 to about 55 percent by weight of the coated granule, and wherein the coating comprises ethylcellulose in an amount from about 10 to about 40 percent by weight of the coated granule, and glyceryl behenate in an amount from about 10 to about 25 percent by weight of the coated granule.

Further provided herein is a dosage form comprising: a matrix, wherein the matrix comprises hydroxypropylmethylcellulose in an amount of about 5 to about 10 percent by weight of the dosage form; and coated granules, wherein the coated granules comprise: a granule comprising hydrocodone in an amount of about 5 to about 35 percent by weight of the granule, ethylcellulose in an amount from about 40 to about 70 percent by weight of the granule, and hydroxypropylmethylcellulose in an amount of about 30 percent by weight of the granule; and a coating on the granule, wherein the coating is present in an amount from about 30 to about 55 percent by weight of the coated granule, and wherein the coating consists essentially of ethylcellulose in an amount from about 10 to about 40 percent by weight of the coated granule, and glyceryl behenate in an amount from about 10 to about 25 percent by weight of the coated granule.

In some embodiments, the release of hydrocodone from a dosage form after 6 hours is less than about 80 percent when tested in 500ml of 0.1 hydrochloric acid using USP dissolution apparatus. In some embodiments, the percent of hydrocodone released after 2 hours in a solution of 0.1N hydrochloric acid and 40% alcohol is no more than 10 percentage points greater than the percent of hydrocodone released in a solution of 0.1N hydrochloric acid in the absence of alcohol. In some embodiments, the release of hydrocodone from the dosage form 30 minutes after simulated oral tampering is less than about 50 percent.

The details of one or more embodiments of the invention are set forth in the accompanying drawings and the description below. Other features, objects, and advantages of the invention will be apparent from the description and drawings, and from the claims.

### FIGURE DESRIPTION

FIG. 1 shows a graph of the Mean (+SD) Plasma Concentration-versus-Time Profiles for Hydrocodone in Healthy Volunteers Administered Single Doses of 45 mg Hydrocodone ER Tablets or 4 x 10 mg Hydrocodone IR Tablets.

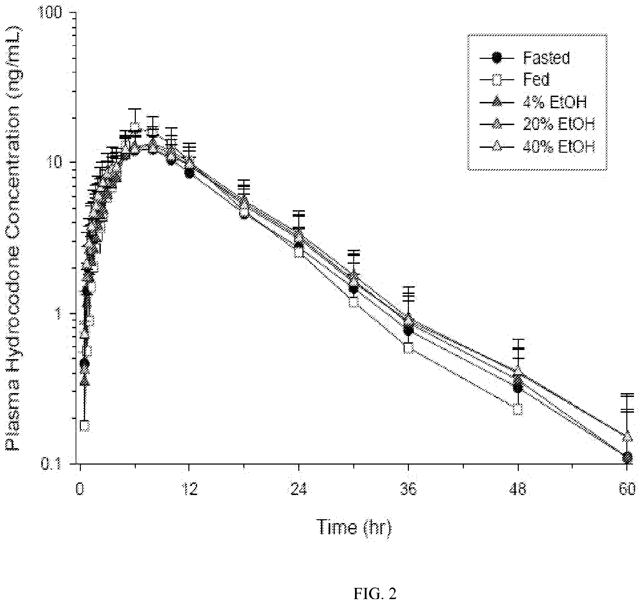

FIG. 2 shows a graph of the Mean (+SD) Plasma Concentration-versus-Time Profiles for Hydrocodone in Healthy Volunteers Administered Single Doses of 15 mg
   Hydrocodone ER Tablets (See Table 3 in the Examples) under Fasted or Fed Conditions or with Ethanol.

### DETAILED DESCRIPTION

Sustained-release oral dosage forms suitable for twice-a-day administration of hydrocodone are provided. The dosage form includes a matrix having a first viscosity modifier and coated granules comprising hydrocodone or a salt form thereof (e.g., hydrocodone bitartrate). In some cases, a dosage form, as described herein, has a release profile such that after 6 hours in 500 ml of 0.1N hydrochloric acid, less than about 80 percent of the hydrocodone is released. In addition, a dosage form may have alcohol and/or crush resistance.

The term "matrix" refers to a monolithic system comprising active substance-containing particles (*e.g.*, coated granules) dispersed and entrapped in a continuum of excipients, *i.e.*, the "matrix forming" substances; *see*, for example, Colombo, P., Santi, P., Siepmann, J., Colombo, G., Sonvico, F., Rossi, A., Luca Strusi, O., 2008. Swellable and Rigid Matrices: Controlled Relelase Matrices with Cellulose Ethers. In: Pharmaceutical Dosage Forms: Tablets, Volume 2: Rational Design and Formulation. Third Edition, Augsburger, L. and Hoag, S. (eds.). Informa Healthcare, New York, London. As set forth further herein, coated granules comprising hydrocodone are dispersed within a described matrix.

Provided herein is a sustained-release oral dosage form including a matrix comprising a first viscosity modifier in an amount from 1 to 10 percent (e.g., about 5 to about 10 percent, including about 6 percent and also including about 10 percent) by weight of the dosage form, and coated granules comprising a granule and a coating on the granule.

The dosage forms described herein can have a release profile such that the release of hydrocodone from the dosage form after 6 hours is less than about 80 percent. In some embodiments, the release of hydrocodone from the dosage form after 10 hours is less than about 85 percent. Release of hydrocodone is measured using the USP dissolution apparatus number 2 and 500 ml of a 0.1 N hydrochloric acid solution as the dissolution medium.

The dosage form may be alcohol resistant. Resistance to alcohol is measured using the USP dissolution apparatus number 2 and 500 ml of a 0.1 N hydrochloric acid solution (normal dissolution) or a 0.1N hydrochloric acid and 40% ethanolic solution (alcohol concentration is 40% v/v; dose dumping dissolution) as the dissolution medium. For an alcohol resistant formulation, as described herein, after 2 hours in a solution of 0.1N hydrochloric acid and 40% ethanol, the percent release of hydrocodone is no more than 10 percentage points greater than the percent of hydrocodone released in the 0.1N hydrochloric acid solution in the absence of alcohol. For example, if the dosage form releases 20% of the hydrocodone in the 0.1N hydrochloric acid solution in the absence of alcohol after 2 hours, then an alcohol resistant dosage form, as described herein, will not release any more than 30% of the hydrocodone in the solution having 0.1N hydrochloric acid and 40% ethanol.

In some embodiments, a dosage form, as described herein, can be crush resistant. Crush resistance is measured using techniques designed to simulate oral tampering. Such methods involve placing a tablet of the dosage form in a ceramic mortar (13 cm outer diameter). A pestle is then used to apply force vertically downward onto the tablet until it breaks. The broken tablet is further crushed using a 360° circular motion with downward force applied throughout. The circular crushing motion is repeated eleven times (twelve strokes total). The resulting powder is transferred to a dissolution vessel to measure *in vitro* drug release. The *in vitro* release profile of the crushed tablet samples is obtained in 500 ml of 0.1N hydrochloric acid dissolution medium. The samples are agitated at 50 rpm using USP apparatus 2 (paddles) at 37 °C. After 30 minutes in the dissolution medium, a crush resistant dosage form exhibits a release of hydrocodone from the dosage form of less than about 50 percent.

The dosage forms described herein exhibit one or more of the above extended release and tamper-resistant characteristics.

A first viscosity modifier, as described herein, is a material, which upon dissolution or dispersion in an aqueous solution or dispersion (e.g., water) at a concentration of 2% w/w (based on the dry material), creates a solution/dispersion with a viscosity of from about 100 to about 200,000 mPa•s (e.g., 4,000 to 175,000 mPa•s, and 75,000 to 140,000 mPa•s) as measured at 20 °C (± 0.2 °C) using the analysis method described in the USP 33 monograph for hypromellose. The first viscosity modifier is selected from the group consisting of sodium alginate, hydroxypropylmethylcellulose, hydroxyethylcellulose, hydroxypropylcellulose, carboxymethylcellulose, sodium carboxymethylcellulose, methylcellulose, polyvinylpyrrolidone, crosslinked polyacrylic acid (e.g., carbomers), gelatin, pectins, gums (e.g., gum arabic, gum tragacanth, xanthan gums, and guar gums), polyethylene oxides, Konjac flour, carrageenan, or mixtures thereof. In some embodiments, the first viscosity modifier is hydroxypropylmethylcellulose. In some embodiments, the first viscosity modifier is a gelling polymer. Examples include hydroxypropylmethylcellulose, hydroxypropylcellulose, methylcellulose, hydroxyethylcellulose, and carboxymethylcellulose. In some embodiments, the gelling polymer is hydroxypropylmethylcellulose (HPMC).

When HPMC is used in the dosage form, the HPMC can have different methyl to hydroxypropyl substitution percent ratios ranging from 30:0 in the A-type, 29:8.5 for the E-type, 28:5 in the F-type, 22:8 for the K-type all available from DOW Chemical Company, Midland, Mich. or any other HPMC polymers available from other suppliers such as Aqualon.

Coated granules of the dosage forms described herein include a granule comprising hydrocodone or a salt form thereof and a coating on the granule. The coated granule comprises a granule comprising hydrocodone or a salt form thereof in an amount from 0.1 to 90 percent by weight of the granule, a first strong film former in an amount from 1 to 90 percent by weight of the granule, a second viscosity modifier in an amount from 1 to 90 percent by weight of the granule, wherein the granule does not contain a fat/wax; and a coating on the granule, wherein the coating is present in an amount from 20 to 80 percent by weight of the coated granule, and wherein the coating comprises a second strong film former in an amount from 10 to 50 percent by weight of the coated granule, and a fat/wax in an amount from 10 to 25 percent by weight of the coated granule.

Hydrocodone can be present in the dosage form as a neutral compound or as a salt form (e.g., hydrocodone bitartrate). As used herein, references to hydrocodone include hydrocodone and salts thereof, especially hydrocodone bitartrate. A person skilled in the art will know how to prepare and select suitable salt forms for example, as described in Handbook of Pharmaceutical Salts: Properties, Selection, and Use By P. H. Stahl and C. G. Wermuth (Wiley-VCH 2002). In some embodiments, the hydrocodone or a salt form thereof is present in an amount from about 1 to about 60 percent by weight of the granule. In some embodiments, the hydrocodone or a salt form thereof is present in an amount from about 1 to about 50 percent by weight of the granule. In some embodiments, the hydrocodone or a salt form thereof is present in an amount from about 5 to about 35 percent by weight of the granule.

A strong film former is a polymer, which is at least slightly soluble, preferably, soluble in alcohol and at most slightly soluble in water and forms a dry 76 µm (3-mil) film with tensile strength not less than 6.895 MPa (1000 lb/in²) when measured by the appropriate tensile strength measuring equipment such as the texture analyzer manufactured by Texture Technologies, Brookfield, Lloyd Instruments, and the like. The first and second strong film formers are selected from ethylcellulose; Ammonio Methacrylate Copolymer, Type B (Eudragit RS); Ammonio Methacrylate Copolymer, Type A (Eudragit RL); Amino Methacrylate Copolymer (Eudragit E); Ethyl Acrylate and Methyl Methacrylate Copolymer Dispersion (Eudragit NE); Methacrylic Acid Copolymer, Type A (Eudragit L); Methacrylic Acid Copolymer, Type B (Eudragit S); and shellac. In some cases, the first and second strong film formers are the same.

In some embodiments, a strong film former is ethylcellulose (EC). Ethylcellulose is an inert, hydrophobic polymer and is essentially tasteless, odorless, colorless, non-caloric, and physiologically inert. There are many types of ethylcellulose which can be used, as long as they meet the other requirements, such as alcohol solubility, discussed herein. The ethylcellulose used can have different ethoxy content such as 48.0-49.5% described as N-type; 49.6-51.5% described as T-type; 50.5-52.5% described as X-type; all available from Aqualon, Hercules Research Center, Wilmington, Del.

The ethylcellulose used can have different molecular weights such as including EC polymers of the N-type that form 5% w/w solution in toluene:ethanol (80:20) that have viscosity ranges of 5.6-8.0 mPa.s (centipoise) described as N7; 8.0-11 mPa.s described as N10; 12-16 mPa.s described as N14; 18-24 mPa.s described as N22; 40-52 mPa.s described as N50; 80-105 mPa.s described as N100. The ethylcellulose used can also include different degrees of substitution of ethoxy groups per anhydroglucose unit, such as 2.65-2.81 for the X-type. N-type has values of 2.46-2.58.

In some embodiments, the first strong film former is present in an amount from about 30 to about 80 percent by weight of the granule. For example, the first strong film former can be present in an amount from about 40 to about 70 percent by weight of the granule. The second strong film former is present in an amount from 10 to 50 percent by weight of the coated granule. In some cases, the second strong film former can be present in an amount from about 10 to about 40 percent by weight of the coated granule.

In some embodiments, a second viscosity modifier is the same as the first viscosity modifier used in the matrix of the dosage form. In some cases, the second viscosity modifier is hydroxypropylmethylcellulose. In some embodiments, the second viscosity modifier is present in an amount from about 10 to about 70 percent by weight of the granule. In some embodiments, the second viscosity modifier is present in an amount from about 15 to about 40 percent by weight of the granule, for example about 30 percent by weight of the granule.

A fat/wax, as used herein, is generally hydrophobic and a solid at room temperature (25 °C.). Fats are fatty acid based compounds generally having a hydrophilic/lipophilic balance (HLB) of about 6 or less (e.g., 4 or less; 2 or less), and also have a melting point of at least 30 °C (e.g., at least 40 °C; at least 50 °C). In one embodiment, the fat has an HLB of about 6 or less and a melting point of at least about 30 °C. In another embodiment, it has an HLB of about 4 or less and a melting point of at least about 40 °C. In another embodiment, the fat has an HLB of about 2 or less and a
melting point of at least 50 °C. Fats, including fatty acids and fatty esters, may be substituted or unsubstituted, saturated or unsaturated. In some cases, they have a chain length of at least about 14. Fatty esters may include fatty acid groups bound to alcohols, glycols, or glycerol. With regard to glyercols, the glycerols may be mono-, di-, and tri-fatty substituted glycerols, or mixtures thereof. Thixotropic fats/waxes can also be used.

Suitable fat ingredients include, without limitation, glycerol fatty esters, fatty glyceride derivatives, waxes and fatty alcohols such as, for example, glyceryl behenate (COMPRITOL®), glycerol palmitostearate (PRECIROL®), stearoyl macroglycerides (GELUCIRE® 50/13). In some embodiments, the fat/wax is glyceryl behenate.

Waxes are very complex and difficult to classify. See Kirk-Othmer, Encyclopedia of Chemical Technology (4th ed. 1998) Vol. 25 pp. 614-26. They often meet the criteria described previously for fats (e.g., HLB of about 6 or less and melting point of at least about 30 °C, HLB of about 4 or less and a melting point of at least about 40 °C, HLB of about 2 or less and a melting point of at least 50 °C), but waxes that do not meet these criteria may also be used. Waxes include, without limitation, insect and animal waxes, vegetable waxes, mineral waxes, petroleum waxes, and synthetic waxes. For example, beeswax, carnauba wax, condelilla wax, montan wax, ouricury wax, rice-bran wax, jojoba wax, microcrystalline wax, cetyl ester wax, anionic emulsifying wax, nonionic emulsifying wax and paraffin wax. In one embodiment, the fat/wax is a fatty acid ester of glycerol. For example, the fatty acid ester of glycerol can be glyceryl behenate.

Fat/waxes used in accordance with the present invention may be used in a molten form. It has been discovered, however, that even when used as a generally solid, non-molten form such as relatively small particles at room temperature, they can provide some, if not all of the advantages as molten materials. Any usable particle size which allows for proper formation of the granules or coating and which provides the desired properties may be used. The fat/wax is present from about 10 to about 25 percent by weight of the coated granule. The fat/wax is present in the coating of the granule but not in the granule.

In some embodiments, the coated granule comprises less than about 10 percent water per weight of the coated granule. For example, the coated granule can have less than about 6 percent water per weight of the coated granule. In some cases, organic solvents may replace the water in the processing of the granules. For example, alcohol, such as ethanol, or acetone may be used.

The term "coating" is meant to encompass a material which substantially surrounds the granules and provides some additional function, such as, without limitation, taste masking, storage stability, reduced reactivity, controlled release, and/or abuse resistance. In some embodiments, the coating is present in an amount from about 30 to about 70 percent by weight of the coated granule. For example, the coating can be present in an amount of about 30 to about 55 percent by weight of the coated granule, including about 35 to about 50 percent, e.g. about 40 percent.

In some embodiments, the sustained-release oral dosage form described herein comprises a matrix, wherein the matrix comprises hydroxypropylmethylcellulose in an amount from about 1 to about 10 percent by weight of the dosage form, for example, from about 5 to about 10 percent by weight, including about 6 percent by weight and including about 10 percent by weight, of the dosage form; and coated granules, wherein the coated granules comprise a granule comprising hydrocodone or a salt form thereof in an amount from about 1 to about 60 percent by weight of the granule, for example, from about 5 to about 35 percent by weight of the granule, ethylcellulose in an amount from about 30 to about 80 percent by weight of the granule, for example, from about 40 to about 70 percent by weight of the granule, hydroxypropylmethylcellulose in an amount from about 10 to about 70 percent by weight of the granule, for example, from about 15 to about 40 percent by weight of the granule, including about 30 percent by weight of the granule, wherein the granule does not contain a fat/wax; and a coating on the granule, wherein the coating is present in an amount from about 30 to about 70 percent by weight of the coated granule, for example, in an amount of about 30 to about 55 percent by weight of the coated granule, including about 35 to about 50 percent, e.g. about 40 percent, and wherein the coating comprises ethylcellulose in an amount from about 10 to about 50 percent by weight of the coated granule or from about 10 to about 40 percent by weight of the coated granule, and glyceryl behenate in an amount from about 10 to about 25 percent by weight of the coated granule.

The coated granules and dosage forms as described herein can be prepared using methods known to those in the art, see, for example, U.S. Publication No. 2008/0311205. In general, hydrocodone or a salt form thereof is formulated into polymer-rich granules onto which a polymeric coat is applied. The coated granules are subsequently mixed with a first viscosity modifier.

In some embodiments, the dosage form may also include at least one other ingredient or excipient in addition to the coated particle and first viscosity modifier in the matrix. The other ingredient or excipient may include, but is not limited to, taste masking agents, binders, fillers, sugars, artificial sweeteners, polymers, flavoring agents, coloring agents, lubricants, glidants, bio- or muco-adhesives, surfactants, buffers, and disintegrants. The amount of any one or more of these ingredients will vary with the amount of coating, granule size, shape of the dosage form, form of the dosage form, number of ingredients used, the particular mixture of ingredients used, the number of dosage forms that will formulate a dose, the amount of hydrocodone per dose and the like. Any combination or amounts are contemplated sufficient to produce a dosage form having the described release profile and/or tamper-resistance provided.

"Taste masking agent(s)" include anything known to be used as a taste masking agents in this art. Examples include Eudragit E-100, ethylcellulose, hydroxypropylmethylcellulose, hydroxypropyl cellulose, methylcellulose, Hydroxyethylcellulose, carboxymethylcellulose, shellac, zein, carbomers, fats, waxes, glycerol mono-, di-, tri-glycerides, Compritol, precirol, gelucires, poloxamers, modified chitosans, carrageenans, cellulose acetate trimellitate, hydroxypropyl methylcellulose phthalate, hydroxypropylmethylcellulose acetate succinate, methacrylic acid copolymers including Eudragit L 100, S 100, L30D-55, polyvinylacetate phthalate (PVAP). Taste masking agents can be used in conventional amounts, for example, in an amount of about 0 to about 50 percent by weight of the total dosage form (e.g., about 5 to about 40 percent by weight of the total dosage form; about 10 to about 30 percent by weight of the total dosage form).
Binders can be used to add cohesiveness to powders and provide the necessary bonding to form granules that can be compressed into hard tablets that have acceptable mechanical strength to withstand subsequent processing or shipping and handling. Examples of binders include acacia, tragacanth, gelatin, starch (both modified or unmodified), cellulose materials such as methylcellulose, ethylcellulose,
hydroxypropylmethylcellulose, hydroxypropylcellulose, hydroxyethylcellulose and sodium carboxy methylcellulose, alginic acids and salts thereof, magnesium aluminum silicate, polyethylene glycol, guar gum, polysaccharide acids, bentonites, sugars, invert sugars, and the like, fats, waxes, polyvinylpyrrolidone, polymethacrylate and other acrylic and vinyl-based polymers. Binders can be used in conventional amounts, for example, in an amount of about 0 to about 50 percent by weight of the total dosage form (e.g., about 2 to about 10 percent by weight of the total dosage form).

Fillers can include mannitol, dextrose, sorbitol, lactose, sucrose, and calcium carbonate. Fillers can be used in conventional amounts, for example, in an amount of about 0 to about 90 percent by weight of the total dosage form (e.g., from about 10 to about 50 percent by weight of the total dosage form). In some embodiments, a filler can be a sugar. For example, sugar, sugar alcohols, ketoses, saccharides, polysaccharides, oligosaccharides and the like, as well as celluloses and modified celluloses.

Sugars may also include direct compression and/or non-direct compression sugars. Non-direct compression sugars include, without limitation, dextrose, mannitol, sorbitol, trehalose, lactose and sucrose. These sugars generally exist as either a direct compression sugar, i.e., a sugar which has been modified to increase its compressibility and/or flow, or a non-direct compression sugar which does not have sufficient flowability and/or compressibility to allow it to be used in high speed processing and multi-tablet presses without some sort of augmentation such as, without limitation, a glidant to increase flow, granulation to increase flow and/or compressibility and the like. While not definitive, sometimes a non-direct compression sugar will have at least about 90% of its particles smaller than about 200 microns, and more preferably 80% smaller than about 150 microns.

The amount of total sugar can range from about 0 to about 90 (e.g., about 5 to about 75; about 10 and 50) by weight of the total dosage form. Other non-carbohydrate diluents and fillers which may be used include, for example, dihydrated or anhydrous dibasic calcium phosphate, tricalcium phosphate, calcium carbonate, anhydrous or hydrated calcium sulphate, and calcium lactate trihydrate. Non-carbohydrate diluents and fillers may be used in an amount of from about 0 to about 90 percent (e.g., from about 5 to about 75 percent; from about 10 to about 50 percent) by weight of the total dosage form.

Artificial sweeteners can include saccharin, aspartame, sucralose, neotame, and acesulfame potassium. Artificial sweeteners may be used in conventional amounts, for example, in an amount ranging from about 0.1 to about 2 percent by weight of the total dosage form.

Flavoring agents can include synthetic flavor oils and flavoring aromatics and/or natural oils, extracts from plants, leaves, flowers, fruits and so forth and combinations thereof. For example, cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Also useful as flavoring agents are vanilla, citrus oil, including lemon, orange, banana, grape, lime and grapefruit, and fruit essences, including apple, pear, peach, strawberry, raspberry, cherry, plum, pineapple, apricot and so forth.

Flavoring agents may be used in conventional amounts, for example, in an amount ranging from about 0.01 to about 3 percent by weight of the dosage form (e.g., from about 0.1 to about 2.5 percent by weight of the dosage form; from about 0.25 to about 2 percent by weight of the dosage form).

Coloring agents can include titanium dioxide, iron oxides such as red or yellow iron oxide, and dyes suitable for food such as those known as FD&C dyes and natural coloring agents such as grape skin extract, beet red powder, beta-carotene, annatto, carmine, turmeric, and paprika. Coloring agents may be used in conventional amounts, for example, in an amount ranging from about 0.001 to about 1% by weight of the total dosage form.

Lubricants can include intrinsic or extrinsic lubricants. Intrinsic lubricants may include magnesium, calcium, zinc salts of stearic acid, hydrogenated and partially hydrogenated vegetable oils, animal fats, polyethylene glycol, polyoxyethylene monostearate, talc, light mineral oils, sodium benzoate, sodium lauryl sulphate, magnesium oxide and the like. Lubricants may be used in conventional amounts, for example, in an amount from about 0.1 to about 5 percent by weight of the dosage form (e.g., from about 0.25 to about 2.5 percent; from about 0.5 to about 2 percent). Some of the compounds referred to as lubricants can also be referred to as fat/waxes, but lubricants are generally used in formulations at lower concentrations than fat/waxes and lubricants are generally used to ease processing rather than impart functionality.

Surfactants can include, without limitation, various grades of the following commercial products: Arlacel®, Tween®, Capmul®, Centrophase®, Cremophor®, Labrafac®, Labrafil®, Labrasol®, Myverol®, Tagat®, and any non-toxic short and medium chain alcohols. Surfactants can be used in conventional amounts, for example, in an amount of about 0.01 to about 5 percent by weight of the dosage form (e.g., in an amount of about 0.1 to about 2 percent).

Buffers can include any weak acid or weak base or, preferably, any buffer system that is not harmful to the gastrointestinal mucosa. These include, but are not limited to, sodium carbonate, potassium carbonate, potassium carbonate, disodium hydrogen phosphate, sodium dihydrogen phosphate, and the equivalent potassium salts. Buffers can be used in conventional amounts, for example, in an amount of about 0.1 to about 10 percent by weight of the dosage form (e.g., from about 1 to about 5 percent).

The dosage form may also contain minor amounts of nontoxic substances such as wetting or emulsifying agents, pH buffering agents and the like, for example, sodium acetate, sorbitan monolaurate, triethanolamine, sodium acetate, triethanolamine oleate, sodium lauryl sulfate, dioctyl sodium sulfosuccinate, polyoxyethylene sorbitan fatty acid esters.

A "dosage form", as used herein, is a tablet, capsule, caplet, sachet, powder or other solid known for the administration of medicines orally. It is generally made from a mixture as defined herein and is generally formed (as in a tablet) into a form for use by a doctor or patient for administration.

Dosage forms may be provided in a range of shapes and sizes. In some embodiments, the dosage form is in a size capable of oral administration and provides a therapeutic amount of hydrocodone. Generally, such dosage forms will be less than 3.8 cm (1.5 inches) in any one direction, more preferably less than 2.54 cm (1 inch) and most preferably less than 1.9 cm (0.75 inch). Shapes include but not limited to round with both flat or convex face, capsule shape (caplets), diamond shape, triangular, rectangular, hexagonal, pentagonal, heart-shaped, animal shaped tablets like rabbits, elephants etc. Dosage forms can be any size and shape, but preferable of a size and shape to avoid crushing or abuse.

Dosage forms, especially tablets, may also be coated to improve the appearance of the dosage form, and also to avoid crushing or abuse.

Dosage forms are formulated to be suitable for twice-a-day administration. The amount of hydrocodone present in the dosage form can vary from about 10 mg to about 90 mg (e.g. 15 mg, 30 mg and 45 mg). The dosage form may be used to manage persistent, moderate-to-severe pain in patients requiring continuous, around-the-clock pain relief for an extended period of time.

In some embodiments, the tablet can have a hardness from about 20 to 200 Newtons.

Tablets can either be manufactured by direct compression, wet granulation, dry granulation followed by coating and tablet compression or any other tablet manufacturing technique. See, e.g., U.S. Pat. Nos. 5,178,878, 5,223,264 and 6,024,981.

In the Examples hereinafter, hydrocodone bitartrate includes 9.1 % water of crystallization.

### EXAMPLES

### Comparative Example 1 Dosages Including FAT/WAX in Core, in Coat, in COAT and Core

**Table 1.**

| Uncoated Granules | |
|---|---|
| Material | % w/w |
| hydrocodone bitartrate | 27.00 |
| hydroxypropylmethylcellulose (K100M) | 20.00 |
| ethylcellulose | 43.00 |
| Compritol (glyceryl behenate) | 10.00 |
| | |

| Coated Granules | |
|---|---|
| Material | % w/w |
| uncoated granules | 80.00 |
| ethylcellulose | 13.33 |
| Compritol (glyceryl behenate) | 6.67 |
| | |

| Dosage Form | |
|---|---|
| Materials | % w/w |
| coated granules | 41.86 |
| hydrocodone bitartrate | 2.25 |
| lactose monohydrate | 45.39 |
| hydroxypropylmethylcellulose (K100M) | 10.00 |
| magnesium stearate | 0.50 |

Granules were manufactured in a high shear granulator where hydrocodone bitartrate, hydroxypropylmethylcellulose, Compritol, and a portion of the ethylcellulose were dry mixed for 2 minutes. Then, a 10% hydro-ethanolic (30:70) solution of the remaining ethylcellulose was slowly added while maintaining the granulator impeller and chopper speeds at pre-selected values to provide enough shear for granule formation and growth. Solution addition was continued until the aforementioned percentage of ethylcellulose was realized. The granules were then milled in an impact mill and finally dried.

The uncoated granules were then coated in a bottom spray fluid bed using a 15% alcoholic suspension of a 2:1 ethylcellulose/Compritol mixture to provide a coat of 20% by weight of the coated granules. Coated granules were mixed with lactose monohydrate, hydrocodone bitartrate and hydroxypropylmethylcellulose in diffusion mixer. Magnesium stearate was added and the mixture was further blended. The amount of coated granules charged into the tablet is based on the actual coated granule content of hydrocodone, it is not based on the theoretical content. The blended mixture was then compressed in a rotary tablet press to form tablets. The 0.95 cm (3/8 inch) round tablets weighed 400 mg and had an average hardness of 95 N.

This tablet formulation is also described in Table 2. Table 2 also contains descriptions of numerous additional formulations (referred to by Lot number in Table 2) and the corresponding results from tests performed to examine crush resistance, and alcohol resistance.

The formulations provided in Table 2 that contain coloring agents were formulated by adding the coloring agent to the matrix prior to compression as follows. The coated granules were mixed with the colorant, lactose monohydrate and hydroxypropylmethylcellulose in a diffusion mixer. Magnesium stearate was added and the mixture was further blended. The amount of coated granules charged into the tablet is based on the actual coated granule content of hydrocodone, it is not based on the theoretical content. The blended mixture was then compressed in a rotary tablet press to form tablets. In some examples the coloring agent was preblended with the lactose, delumped, screened, and then mixed with the remaining ingredients prior to compression

**Table 2.**

| **LOT#** | **Tablet Description** | **%R0.5 N** | **%R6 N** | **%R1 10N** | **%R1 2N** | **%R0 .5C** | **%R 2E** | **%R2 N** |
|---|---|---|---|---|---|---|---|---|
| **Comparative Example 4422-14** **45 mg** | Granules: hpmc 20.0 %, EC 43.0 %, Compritol 10.0%, | **12** | **73** | **95** | **101** | **81** | **28** | **36** |
| | Hydrocodone Bitartrate 27.0 % | | | | | | | |
| | 20% Coat: 15 % EC/Compritol (2:1) | | | | | | | |
| | 95 N 400 mg 3/8" round Tablets: 100% Hydrocodone | | | | | | | |
| | Bitartrate 2.25%, Coated granules 41.86%, Lactose | | | | | | | |
| | 45.39%, HPMC 10.00%, Magnesium Stearate 0.5%. | | | | | | | |
| | Tablet Hardness ave. 95N. | | | | | | | |
| **Comparative Example 4422-16** **45 mg** | Granules: hpmc 20.0 %, EC 43.0 %, Compritol | **8** | **51** | **72** | **80** | **55** | **24** | **22** |
| | 10.0%, Hydrocodone Bitartrate 27.0 % | | | | | | | |
| | 30% Coat: 15 % EC/Compritol (2:1) | | | | | | | |
| | 73 N 400 mg 3/8" round Tablets: 100% | | | | | | | |
| | Hydrocodone Bitartrate 2.25%, Coated granules 47.62%, | | | | | | | |
| | Lactose 39.63%, HPMC 10.00%, Magnesium Stearate 0.5%. | | | | | | | |
| | Tablet Hardness ave. 73N. | | | | | | | |
| **Comparative Example 4422-18** **45 mg** | Granules: hpmc 20.0 %, EC 43.0 %, Compritol | **10** | **67** | **85** | **91** | **81** | **28** | **33** |
| | 10.0%, Hydrocodone Bitartrate 27.0 % | | | | | | | |
| | 20% Coat: 15 % EC/Compritol (2:1) | | | | | | | |
| | 85 N 400 mg 3/8" round Tablets: Uncoated | | | | | | | |
| | Granules 8.30%, Coated granules 41.86%, Lactose | | | | | | | |
| | 39.34%, HPMC 10.00%, Magnesium Stearate 0.5%. | | | | | | | |
| | Tablet Hardness ave. 85N. | | | | | | | |
| **Comparative Example 4422-20** **45 mg** | Granules: hpmc 20.0 %, EC 43.0 %, Compritol | **8** | **48** | **69** | **76** | **53** | **24** | **21** |
| | 10.0%, Hydrocodone Bitartrate 27.0 % | | | | | | | |
| | 30% Coat: 15 % EC/Compritol (2:1) | | | | | | | |
| | 67 N 400 mg 3/8" round Tablets: Uncoated | | | | | | | |
| | Granules 8.30%, Coated granules 47.62%, Lactose | | | | | | | |
| | 33.58%, HPMC 10.00%, Magnesium Stearate 0.5%. | | | | | | | |
| | Tablet Hardness ave. 67N. | | | | | | | |
| **Comparative Example 4422-42** **45 mg** | Granules: hpmc 20.0 %, EC 43.0 %, Compritol | **14** | **73** | **91** | **96** | **84** | **31** | **39** |
| | 10.0%, Hydrocodone Bitartrate 27.0 % | | | | | | | |
| | 20% Coat: 15 % EC/Compritol (2:1) | | | | | | | |
| | 87 N 400 mg 3/8" round Tablets: Hydrocodone | | | | | | | |
| | Bitartrate 2.25%, Coated granules 43.06%, Lactose | | | | | | | |
| | 44.19%, HPMC 10.00%, Magnesium Stearate 0.5%. | | | | | | | |
| | Tablet Hardness ave. 87N. | | | | | | | |
| **Comparative Example 4422-44** **45 mg** | Granules: hpmc 20.0 %, EC 43.0 %, Compritol | **12** | **71** | **91** | **97** | **84** | **28** | **37** |
| | 10.0%, Hydrocodone Bitartrate 27.0 % | | | | | | | |
| | 20% Coat: 15 % EC/Compritol (2:1) | | | | | | | |
| | 66 N 400 mg 3/8" round Tablets: Coated | | | | | | | |
| | granules 53.83%, Lactose 35.67%, HPMC 10.00%, | | | | | | | |
| | Magnesium Stearate 0.5%. | | | | | | | |
| | Tablet Hardness ave.66N. | | | | | | | |
| **Comparative Example 4422-46** **45 mg** | Granules: hpmc 20.0 %, EC 43.0 %, Compritol | **7** | **55** | **76** | **84** | **62** | **27** | **23** |
| | 10.0%, Hydrocodone Bitartrate 27.0 % | | | | | | | |
| | 30% Coat: 15 % EC/Compritol (2:1) | | | | | | | |
| | 53 N 400 mg 3/8" round Tablets: Coated | | | | | | | |
| | granules 58.60%, Lactose 30.91%, HPMC 10.00%, Magnesium Stearate 0.5%. | | | | | | | |
| | Tablet Hardness ave. 53N. | | | | | | | |
| **Comparative Example 4422-48** **45 mg** | Granules: hpmc 30.0%, EC 43.0 %, | **14** | **71** | **90** | **96** | **85** | **28** | **38** |
| | Hydrocodone Bitartrate 27.0 % | | | | | | | |
| | 20% Coat: 15 % EC/Compritol (2:1) | | | | | | | |
| | 86 N 400 mg 3/8" round Tablets: 100.0% | | | | | | | |
| | Hydrocodone Bitartrate 2.25%, Coated granules 44.12%, Lactose 43.13%, HPMC 10.00%, Magnesium Stearate 0.5%. | | | | | | | |
| | Tablet Hardness ave. 86N. | | | | | | | |
| **4422-50** **45 mg** | Granules: hpmc 30.0%, EC 43.0 %, | **10** | **63** | **81** | **86** | **69** | **27** | **31** |
| | Hydrocodone Bitartrate 27.0 % | | | | | | | |
| | 30% Coat: 15 % EC/Compritol (2:1) | | | | | | | |
| | 73 N 400 mg 3/8" round Tablets: 100.0% | | | | | | | |
| | Hydrocodone Bitartrate 2.25%, Coated granules 49.45%, | | | | | | | |
| | Lactose 37.80%, HPMC 10.00%, Magnesium Stearate 0.5%. | | | | | | | |
| | Tablet Hardness ave. 73N. | | | | | | | |
| **Comparative Example 4422-52** **45 mg** | Granules: hpmc 30.0%, EC 43.0 %, Hydrocodone | **15** | **71** | **88** | **92** | **87** | **26** | **39** |
| | Bitartrate 27.0 % | | | | | | | |
| | 20% Coat: 15 % EC/Compritol (2:1) | | | | | | | |
| | 70 N 400 mg 3/8" round Tablets: Coated granules | | | | | | | |
| | 55.15%, Lactose 34.35%, HPMC 10.00%, Magnesium | | | | | | | |
| | Stearate 0.5%. | | | | | | | |
| | Tablet Hardness ave. 70N. | | | | | | | |
| **4422-54** **45 mg** | Granules: hpmc 30.0%, EC 43.0 %, Hydrocodone | **8** | **60** | **79** | **84** | **56** | **27** | **27** |
| | Bitartrate 27.0 % | | | | | | | |
| | 30% Coat: 15 % EC/Compritol (2:1) | | | | | | | |
| | 51 N 400 mg 3/8" round Tablets: Coated granules | | | | | | | |
| | 61.81%, Lactose 27.69%, HPMC 10.00%, Magnesium | | | | | | | |
| | Stearate 0.5%. | | | | | | | |
| | Tablet Hardness ave. 51N. | | | | | | | |
| **Comparative Example 4422-56** **15 mg** | Granules: hpmc 30.0%, EC 43.0 %, Hydrocodone | **18** | **80** | **97** | **101** | **96** | **38** | **47** |
| | Bitartrate 27.0 % | | | | | | | |
| | 20% Coat: 15 % EC/Compritol (2:1) | | | | | | | |
| | 126 N 400 mg 3/8" round Tablets: 100% | | | | | | | |
| | Hydrocodone Bitartrate 0.72%, Coated granules 17.60%, Lactose 71.62%, HPMC 9.58%, Magnesium Stearate 0.48%. | | | | | | | |
| | Tablet Hardness ave. 126N. | | | | | | | |
| **Comparative Example 4422-58** **15 mg** | Granules: hpmc 20.0%, EC 43.0 %, | **14** | **70** | **96** | **96** | **72** | **37** | **35** |
| | Hydrocodone Bitartrate 27.0 %, Compritol 10.0% | | | | | | | |
| | 30% Coat: 15 % EC/Compritol (2:1) | | | | | | | |
| | 111 N 400 mg 3/8" round Tablets: Coated | | | | | | | |
| | granules 19.53%, Lactose 69.97%, HPMC 10.00%, | | | | | | | |
| | Magnesium Stearate 0.50%. | | | | | | | |
| | Tablet Hardness ave. 111N. | | | | | | | |
| **4422-68** **45 mg** | Granules: hpmc 30.0%, EC 43.0 %, | **9** | **59** | **80** | **85** | **59** | **25** | **26** |
| | Hydrocodone Bitartrate 27.0 % | | | | | | | |
| | 30% Coat: 15 % EC/Compritol (2:1) | | | | | | | |
| | 95 N 575 mg Capsule Shaped Tablets: Coated | | | | | | | |
| | granules 44.22%, Lactose 43.78%, HPMC 10.00%, | | | | | | | |
| | Magnesium Stearate 2.00%. | | | | | | | |
| | Tablet Hardness ave. 95N. | | | | | | | |
| **4422-70** | Granules: hpmc 30.0%, EC 43.0 %, | **4** | **49** | **72** | **80** | **36** | **23** | **17** |
| **45 mg** | Hydrocodone Bitartrate 27.0 % | | | | | | | |
| | 40% Coat: 15 % EC/Compritol (2:1) | | | | | | | |
| | 88 N 575 mg Capsule Shaped Tablets: Coated | | | | | | | |
| | granules 49.53%, Lactose 38.47%, HPMC 10.00%, Magnesium Stearate 2.00%. | | | | | | | |
| | Tablet Hardness ave. 88N. | | | | | | | |
| **4422-76** **45 mg** | Granules: hpmc 30.0%, EC 43.0 %, | **5** | **58** | **80** | **87** | **42** | **24** | **21** |
| | Hydrocodone Bitartrate 27.0 % | | | | | | | |
| | 40% Coat: 15 % EC/Compritol (2:1) | | | | | | | |
| | 123 N 575 mg Capsule Shaped Tablets Coated | | | | | | | |
| | granules 49.53%, Lactose 43.97%, HPMC 6.00%, Magnesium Stearate 0.50%. | | | | | | | |
| | Tablet Hardness ave. 123N. | | | | | | | |
| **4422-78** **45 mg** | Granules: hpmc 30.0%, EC 43.0 %, | **9** | **74** | **86** | **88** | **41** | **29** | **35** |
| | Hydrocodone Bitartrate 27.0 % | | | | | | | |
| | 40% Coat: 15 % EC/Compritol (2:1) | | | | | | | |
| | 139 N 575 mg Capsule Shaped Tablets Coated | | | | | | | |
| | granules 49.53%, Lactose 47.97%, HPMC 2.00%, | | | | | | | |
| | Magnesium Stearate 0.50%. | | | | | | | |
| | Tablet Hardness ave. 139N. | | | | | | | |
| **4422-89** | Granules: hpmc 30.0%, EC 43.0 %, | **6** | **69** | **87** | **90** | **49** | **25** | **26** |
| **45 mg** | Hydrocodone Bitartrate 27.0 % | | | | | | | |
| | 40% Coat: 15 % EC/Compritol (2:1) | | | | | | | |
| | 112 N 575 mg Capsule Shaped Tablets Coated | | | | | | | |
| | granules 49.53%, Lactose 39.97%, HPMC K100LV | | | | | | | |
| | 10.00%, Magnesium Stearate 0.50%. | | | | | | | |
| | Tablet Hardness ave. 112N. | | | | | | | |
| **4422-91** **45 mg** | Granules: hpmc 30.0%, EC 43.0 %, | **7** | **42** | **58** | **64** | **41** | **21** | **19** |
| | Hydrocodone Bitartrate 27.0 % | | | | | | | |
| | 50% Coat: 15 % EC/Compritol (2:1) | | | | | | | |
| | 118 N 575 mg Capsule Shaped Tablets Coated | | | | | | | |
| | granules 51.83%, Lactose 41.67%, HPMC 6.00%, | | | | | | | |
| | Magnesium Stearate 0.50%. | | | | | | | |
| | Tablet Hardness ave. 118N. | | | | | | | |
| **4422-93** **15 mg** | Granules: hpmc 30.0%, EC 43.0 %, | **6** | **38** | **53** | **58** | **47** | **25** | **18** |
| | Hydrocodone Bitartrate 27.0 % | | | | | | | |
| | 50% Coat: 15 % EC/Compritol (2:1) | | | | | | | |
| | 106 N 400 mg 3/8" round Tablets Coated | | | | | | | |
| | granules 24.83%, Lactose 64.67%, HPMC 10.00%, | | | | | | | |
| | Magnesium Stearate 0.50%. | | | | | | | |
| | Tablet Hardness ave. 106N. | | | | | | | |
| **4422-95** | Granules: hpmc 30.0%, EC 43.0 %, | **10** | **50** | **62** | **67** | **49** | **36** | **27** |
| **15 mg** | Hydrocodone Bitartrate 27.0 % | | | | | | | |
| | 50% Coat: 15 % EC/Compritol (2:1) | | | | | | | |
| | 120 N 400 mg 3/8" round Tablets Coated | | | | | | | |
| | granules 24.83%, Lactose 68.67%, HPMC 6.00%, | | | | | | | |
| | Magnesium Stearate 0.50%. | | | | | | | |
| | Tablet Hardness ave. 120N. | | | | | | | |
| **4601-1** **45 mg** | Granules: hpmc 30.0%, EC 43.0 %, | **9** | **71** | **90** | **95** | **63** | **28** | **34** |
| | Hydrocodone Bitartrate 27.0 % | | | | | | | |
| | 35% Coat: 15 % EC/Compritol (2:1) | | | | | | | |
| | 139 N 575 mg Capsule Shaped Tablets Coated | | | | | | | |
| | granules 46.86%, Lactose 46.64%, HPMC 6.00%, | | | | | | | |
| | Magnesium Stearate 0.50%. | | | | | | | |
| | Tablet Hardness ave. 139N. | | | | | | | |
| **4601-3** **15 mg** | Granules: hpmc 30.0%, EC 43.0 %, | **21** | **62** | **72** | **76** | **56** | **53** | **40** |
| | Hydrocodone Bitartrate 27.0 % | | | | | | | |
| | 50% Coat: 15 % EC/Compritol (2:1) | | | | | | | |
| | 164 N 575 mg Capsule Shaped Tablets Coated | | | | | | | |
| | granules 17.28%, Lactose 51.22%, Microcrystalline | | | | | | | |
| | Cellulose 25.00, HPMC 6.00%, Magnesium Stearate | | | | | | | |
| | 0.50%. | | | | | | | |
| | Tablet Hardness ave. 164N. | | | | | | | |
| **4601-16** **15 mg** | Granules: hpmc 30.0%, EC 43.0 %, | **6** | **64** | **85** | **90** | **53** | **37** | **25** |
| | Hydrocodone Bitartrate 27.0 % | | | | | | | |
| | 45% Coat: 15 % EC/Compritol (2:1) | | | | | | | |
| | 164 N 575 mg Capsule Shaped Tablets Coated | | | | | | | |
| | granules 18.37%, Lactose 71.13%, HPMC 10.00%, | | | | | | | |
| | Magnesium Stearate 0.50%. | | | | | | | |
| | Tablet Hardness ave. 164N. | | | | | | | |
| **4601-18** **15 mg** | Granules: hpmc 30.0%, EC 43.0 %, | **7** | **61** | **82** | **88** | **50** | **39** | **25** |
| | Hydrocodone Bitartrate 27.0 % | | | | | | | |
| | 40% Coat: 15 % EC/Compritol (2:1) | | | | | | | |
| | 161 N 575 mg Capsule Shaped Tablets Coated | | | | | | | |
| | granules 16.72%, Lactose 72.78%, HPMC 10.00%, | | | | | | | |
| | Magnesium Stearate 0.50%. | | | | | | | |
| | Tablet Hardness ave. 161N. | | | | | | | |
| **4601-20** **45 mg** | Granules: hpmc 30.0%, EC 43.0 %, | **4** | **51** | **75** | **82** | **31** | **23** | **16** |
| | Hydrocodone Bitartrate 27.0 % | | | | | | | |
| | 45% Coat: 15 % EC/Compritol (2:1) | | | | | | | |
| | 137 N 575 mg Capsule Shaped Tablets Coated | | | | | | | |
| | granules 55.11%, Lactose 38.29%, HPMC 6.00%, Red | | | | | | | |
| | Iron Oxide 0.1%, Magnesium Stearate 0.50%. | | | | | | | |
| | Tablet Hardness ave. 137N. | | | | | | | |
| | | | | | | | | |
| **4601-22** **45 mg** | Granules: hpmc 30.0%, EC 43.0 %, | **5** | **51** | **74** | **82** | **36** | **25** | **18** |
| | Hydrocodone Bitartrate 27.0 % | | | | | | | |
| | 40% Coat: 15 % EC/Compritol (2:1) | | | | | | | |
| | 117 N 575 mg Capsule Shaped Tablets Coated | | | | | | | |
| | granules 50.17%, Lactose 43.23%, HPMC 6.00%, Red | | | | | | | |
| | Iron Oxide 0.10%, Magnesium Stearate 0.50%. | | | | | | | |
| | Tablet Hardness ave. 117N. | | | | | | | |
| **4601-82** **15 mg** | Granules: hpmc 30.0%, EC 61.0 %, | **7** | **64** | **81** | **85** | **46** | **26** | **29** |
| | Hydrocodone Bitartrate 9.0 % | | | | | | | |
| | 42.5% Coat: 15 % EC/Compritol (2:1) | | | | | | | |
| | 126 N 575 mg Capsule Shaped Tablets Coated | | | | | | | |
| | granules 54.35%, Lactose 39.15%, HPMC 6.00%, | | | | | | | |
| | Magnesium Stearate 0.50%. | | | | | | | |
| | Tablet Hardness ave. 126N. | | | | | | | |
| **4601-84** **15 mg** | Granules: hpmc 30.0%, EC 61.0 %, | **5** | **55** | **74** | **79** | **41** | **22** | **23** |
| | Hydrocodone Bitartrate 9.0 % | | | | | | | |
| | 42.5% Coat: 15 % EC/Compritol (2:1) | | | | | | | |
| | 125 N 575 mg Capsule Shaped Tablets Coated | | | | | | | |
| | granules 54.35%, Lactose 35.15%, HPMC 10.00%, | | | | | | | |
| | Magnesium Stearate 0.50%. Tablet Hardness ave. 125N. | | | | | | | |
| **4828-49*** **15 mg** | Granules: hpmc 30.0%, EC 61.0 %, | **4** | **51** | **71** | **77** | **44** | **21** | **19** |
| | Hydrocodone Bitartrate 9.0 % | | | | | | | |
| | 42.5% Coat: 15 % EC/Compritol (2:1) | | | | | | | |
| | 137 N 575 mg Capsule Shaped Tablets Coated | | | | | | | |
| | granules 54.35%, Lactose 35.05%, HPMC 10.00%, Red | | | | | | | |
| | Iron Oxide 0.1%, Magnesium Stearate 0.50%. | | | | | | | |
| | Tablet Hardness ave. 137N. | | | | | | | |
| **4828-53*** **15 mg** | Granules: hpmc 30.0%, EC 43.0 %, | **5** | **67** | **89** | **94** | **49** | **35** | **27** |
| | Hydrocodone Bitartrate 27.0 % | | | | | | | |
| | 42.5% Coat: 15 % EC/Compritol (2:1) | | | | | | | |
| | 192 N 575 mg Capsule Shaped Tablets Coated | | | | | | | |
| | granules 17.28%, Lactose 72.12%, HPMC 10.00%, Red | | | | | | | |
| | Iron Oxide 0.1%, Magnesium Stearate 0.50%. | | | | | | | |
| | Tablet Hardness ave. 192N. | | | | | | | |
| **4828-56*** **30 mg** | Granules: hpmc 30.0%, EC 43.0 %, | **7** | **76** | **94** | **97** | **41** | **35** | **35** |
| | Hydrocodone Bitartrate 27.0 % | | | | | | | |
| | 42.5% Coat: 15 % EC/Compritol (2:1) | | | | | | | |
| | 151N 575 mg Capsule Shaped Tablets Coated | | | | | | | |
| | granules 34.55%, Lactose 58.85%, HPMC 6.00%, | | | | | | | |
| | Yellow Iron Oxide 0.1%, Magnesium Stearate 0.50%. | | | | | | | |
| | Tablet Hardness ave. 151N. | | | | | | | |
| **200904** **45 mg** | Granules: hpmc 30.0%, EC 43.0 %, | **3** | **40** | **63** | **70** | **18** | **20** | **11** |
| | Hydrocodone Bitartrate 27.0 % | | | | | | | |
| | 50% Coat: 15 % EC/Compritol (2:1) | | | | | | | |
| | 68N 575 mg Capsule Shaped Tablets Coated | | | | | | | |
| | granules 57.97%, Lactose 35.53%, HPMC 6.00%, | | | | | | | |
| | Magnesium Stearate 0.50%. | | | | | | | |
| | Tablet Hardness ave. 68N. | | | | | | | |
| **200905** **45 mg** | **Granules: hpmc 30.0%, EC 43.0 %,** | **4** | **55** | **80** | **86** | **32** | **24** | **18** |
| | **Hydrocodone Bitartrate 27.0 %** | | | | | | | |
| | **42.5% Coat: 15 % EC/Compritol (2:1)** | | | | | | | |
| | **87N 575 mg Capsule Shaped Tablets Coated** | | | | | | | |
| | **granules 50.49%, Lactose 43.01%, HPMC 6.00%,** | | | | | | | |
| | **Magnesium Stearate 0.50%.** | | | | | | | |
| | **Tablet Hardness ave. 87N.** | | | | | | | |
| | **See also pK study results provided herein.** | | | | | | | |
| **200906** **45 mg** | Granules: hpmc 30.0%, EC 43.0 %, | **8** | **68** | **88** | **93** | **57** | **27** | **31** |
| | Hydrocodone Bitartrate 27.0 % | | | | | | | |
| | 35% Coat: 15 % EC/Compritol (2:1) | | | | | | | |
| | 101N 575 mg Capsule Shaped Tablets Coated | | | | | | | |
| | granules 44.47%, Lactose 49.03%, HPMC 6.00%, | | | | | | | |
| | Magnesium Stearate 0.50%. | | | | | | | |
| | Tablet Hardness ave. 101N. | | | | | | | |
| **200922*** **15 mg** | Granules: hpmc 30.0%, EC 61.0 %, | **5** | **41** | **60** | **66** | **30** | **20** | **15** |
| | Hydrocodone Bitartrate 9.0 % | | | | | | | |
| | 42.5% Coat: 15 % EC/Compritol (2:1) | | | | | | | |
| | 95 N 575 mg Capsule Shaped Tablets Coated | | | | | | | |
| | granules 50.17%, Lactose 39.23%, HPMC 10.00%, Red | | | | | | | |
| | Iron Oxide 0.1%, Magnesium Stearate 0.50%. | | | | | | | |
| | Tablet Hardness ave. 95N. | | | | | | | |
| **200923*** **15 mg** | Granules: hpmc 30.0%, EC 43.0%, Hydrocodone | **5** | **56** | **76** | **82** | **48** | **34** | **21** |
| | Bitartrate 27.0 % | | | | | | | |
| | 42.5% Coat: 15 % EC/Compritol (2:1) | | | | | | | |
| | 139 N 575 mg Capsule Shaped Tablets Coated | | | | | | | |
| | granules 16.83%, Lactose 72.57%, HPMC 10.00%, Red | | | | | | | |
| | Iron Oxide 0.1%, Magnesium Stearate 0.50%. | | | | | | | |
| | Tablet Hardness ave. 139N. | | | | | | | |
| **C56593** **45 mg** | Granules: hpmc 30.0%, EC 43.0 %, | **6** | **44** | **63** | **70** | **29** | **26** | **18** |
| | Hydrocodone Bitartrate 27.0 % | | | | | | | |
| | 42.5% Coat: 15 % EC/Compritol (2:1) | | | | | | | |
| | 68N 575 mg Capsule Shaped Tablets Coated | | | | | | | |
| | granules 50.49%, Lactose 43.01%, HPMC 6.00%, | | | | | | | |
| | Magnesium Stearate 0.50%. | | | | | | | |
| | Tablet Hardness ave. 68N. | | | | | | | |
| **C63778** **15 mg** | Granules: hpmc 30.0%, EC 43.0 %, | **9** | **67** | **87** | **93** | **41** | **25** | **31** |
| | Hydrocodone Bitartrate 27.0 % | | | | | | | |
| | 40% Coat: 15 % EC/Compritol (2:1) | | | | | | | |
| | 575 mg Capsule Shaped Tablets Coated granules | | | | | | | |
| | 16.10%, Lactose 73.30%, HPMC 10.00%, red iron oxide | | | | | | | |
| | 0.01, Magnesium Stearate 0.50%. | | | | | | | |
| | Tablet Hardness ave. 203N. | | | | | | | |
| **C63780** **30 mg** | Granules: hpmc 30.0%, EC 43.0 %, | **7** | **59** | **81** | **89** | **32** | **22** | **25** |
| | Hydrocodone Bitartrate 27.0 % | | | | | | | |
| | 40% Coat: 15 % EC/Compritol (2:1) | | | | | | | |
| | 575 mg Capsule Shaped Tablets Coated granules | | | | | | | |
| | 32.21%, Lactose 57.19%, HPMC 10.00%, yellow iron | | | | | | | |
| | oxide 0.01, Magnesium Stearate 0.50%. | | | | | | | |
| | Tablet Hardness ave. 164N. | | | | | | | |
| **C63784** **45 mg** | Granules: hpmc 30.0%, EC 43.0 %, | **8** | **60** | **79** | **85** | **25** | **21** | **28** |
| | Hydrocodone Bitartrate 27.0 % | | | | | | | |
| | 40% Coat: 15 % EC/Compritol (2:1) | | | | | | | |
| | 575 mg Capsule Shaped Tablets Coated granules | | | | | | | |
| | 48.31%, Lactose 45.19%, HPMC 6.00%, Magnesium Stearate 0.50%. | | | | | | | |
| | Tablet Hardness ave. 113N. | | | | | | | |
| **C63781** **60 mg** | Granules: hpmc 30.0%, EC 43.0 %, | **13** | **59** | **76** | **82** | **28** | **22** | **32** |
| | Hydrocodone Bitartrate 27.0 % | | | | | | | |
| | 40% Coat: 15 % EC/Compritol (2:1) | | | | | | | |
| | 1150 mg Capsule Shaped Tablets Coated | | | | | | | |
| | granules 32.21%, Lactose 61.19%, HPMC 6.00%, | | | | | | | |
| | FD&C blue #2 aluminum lake 01.0, Magnesium Stearate | | | | | | | |
| | 0.50%. Tablet Hardness ave. 239N. | | | | | | | |
| **C63786** **90 mg** | Granules: hpmc 30.0%, EC 43.0 %, | **8** | **49** | **67** | **75** | **22** | **18** | **24** |
| | Hydrocodone Bitartrate 27.0 % | | | | | | | |
| | 40% Coat: 15 % EC/Compritol (2:1) | | | | | | | |
| | 1150 mg Capsule Shaped Tablets Coated | | | | | | | |
| | granules 48.31%, Lactose 44.99%, HPMC 6.00%, | | | | | | | |
| | FD&C blue #2 aluminum lake 01.0, yellow iron oxide | | | | | | | |
| | 0.10, Magnesium Stearate 0.50%. Tablet Hardness ave. | | | | | | | |
| | 203N. | | | | | | | |

| | | | | | | | | |
|---|---|---|---|---|---|---|---|---|
| %R0.5N = percent release in 0.5 hours normal conditions (e.g., no crushing or ethanol exposure); %R6N = percent release in 6 hours normal conditions (e.g., no crushing or ethanol exposure); %R10N= percent release in 10 hours normal conditions (e.g., no crushing or ethanol exposure); %R12N= percent release in 12 hours normal conditions (e.g., no crushing or ethanol exposure) %R0.5C= percent release in 0.5 hours crushing conditions; %R2E = percent release in 2 hours ethanol exposure; %R2N = percent release in 2 hours normal conditions (e.g., no crushing or ethanol exposure) * For these formulations, the blending process was conducted as follows: Colorant and lactose monohydrate were blended in a diffusion mixer, de-lumped using a screening mill with rotating impeller and re-blended in a diffusion mixer. The pre-blend was then mixed with coated granules and hydroxypropylmethylcellulose in a diffusion blender. Magnesium stearate was added and the mixture was further blended. | | | | | | | | |

### Example 2 - Dissolution rates and tamper resistance

Dissolution in 0.1 N hydrochloric acid, 0.1 N hydrochloric acid and 40% v/v alcohol, and simulated oral tampering of various formulations disclosed herein were tested. Tablets were tested using the USP dissolution apparatus number 2 using 500 ml of 0.1 N hydrochloric acid (normal dissolution) or 40% ethanolic solution (dose dumping dissolution) as the dissolution medium. Unless otherwise specified, aliquots were removed after 60, 120, 240, 480, 720, 960, 1200, and 1440 minutes of stirring in the normal dissolution test and after 15, 30, 45, 60, 120, 180, 240, and 360 minutes for the dose dumping dissolution. Samples were analyzed for hydrocodone using HPLC.

Simulated oral tampering testing was conducted by crushing the tablets using ceramic mortars and pestles. A tablet is placed in a ceramic mortar (13 cm outer diameter). A pestle is used to apply force vertically downward onto the tablet until it breaks. The broken tablet is further crushed using a 360° circular motion with downward force applied throughout. The circular crushing motion is repeated eleven times (twelve strokes total). The resulting powder is transferred to a dissolution vessel for in vitro drug release. The in vitro release profile of the crushed tablet samples is obtained in 500 mL of 0.1 N hydrochloric acid dissolution medium. The samples are agitated at 50 rpm with USP apparatus 2 (paddles) at 37°C. These are the same in vitro conditions as those employed in the in vitro dissolution test described above. Unless otherwise specified, aliquots are removed after 15, 30, 45, 60, and 120 minutes of stirring and are analyzed for hydrocodone using HPLC.

Results of the above experiments are detailed in Table 2. As one of ordinary skill in the art will appreciate the degree of crush resistance exhibited by a particular formulation depends upon the composition of the formulation. To compare the relative crush resistance between two formulations the difference in % release exhibited at a given time point for a first formulation can be compared to a second formulation, for example with reference to Table 2, formulation (1) had an 81 % release rate at .5 hours after crushing, and formulation (2) had a 55% release rate after crushing at .5 hours. When comparing these two formulations one could characterize formulation (2) as having 26% more crush resistance (e.g., the difference between the % released in 0.5 hours after crushing from formulation 1 and the % release in 0.5 hours after crushing formulation (2) . One could also characterize formulation (2) as having 20% or 25% better crush resistance.

### Example 3. Pharmacokinetic Study (PK Study)

This was a Phase 1, single-center, randomized, open-label, 4-period crossover study in healthy male and female volunteers to characterize the pharmacokinetics of 3 prototypes of 45 mg hydrocodone bitartrate extended release (ER) tablet (Treatments A, B, and C) and a commercially available hydrocodone bitartrate/acetaminophen immediate-release (IR) tablet (Treatment D).

Subjects (n=40) were randomly assigned to 1 of 4 treatment sequences: ABCD, BCDA, CDAB, or DABC, whereby A was a 45-mg hydrocodone bitartrate ER tablet prepared according to an example having coated granules with 35% coat level, B was a 45-mg hydrocodone bitartrate ER tablet prepared according to LOT 200905 shown in bold in Table 2, above (coated granules with 42.5% coat level), C was a 45-mg hydrocodone bitartrate ER tablet prepared according to an example having coated granules with 50% coat level, and D was one 10-mg/325-mg hydrocodone bitartrate/acetaminophen IR tablet (commercially available NORCO) dosed every 6 hours until 4 tablets had been administered.

Hydrocodone was administered to the subjects under fasting conditions. Subjects were to receive each treatment during the study, with a minimum 5-day washout between dosing sessions. Subjects also received one 50-mg tablet of naltrexone for blockade of opioid effects approximately 15 hours and 3 hours before and approximately 9 hours and 21 hours after study drug administration in each treatment period. Venous blood samples were collected by venipuncture or indwelling catheter immediately before hydrocodone administration and through 72 hours post dose to characterize the pharmacokinetics of hydrocodone and hydromorphone (an active metabolite). Samples were collected immediately before and 15, 30, and 45 minutes, and 1, 1.25, 1.5, 1.75, 2, 2.25, 2.5, 3, 3.5, 4, 5, 6, 8, 10, 12, 18, 24, 30, 36, 48, 60, and 72 hours after administration of Treatments A, B, and C. For Treatment D, samples were collected immediately before and 15, 30, and 45 minutes, and 1, 1.25, 1.5, 1.75, 2, 2.25, 2.5, 3, 3.5, 4, 5, 6, 7, 7.25, 7.5, 12, 13, 13.25, 13.5, 18, 18.25, 18.5, 18.75, 19, 19.25, 19.5, 19.75, 20, 20.25, 20.5, 21, 21.5, 22, 23, 24, 30, 36, 48, 60, and 72 hours after the initial drug administration.

Concentrations of hydrocodone and hydromorphone were determined in human plasma samples using a validated high-performance liquid chromatography method with tandem mass spectrometric detection (LC-MS/MS).

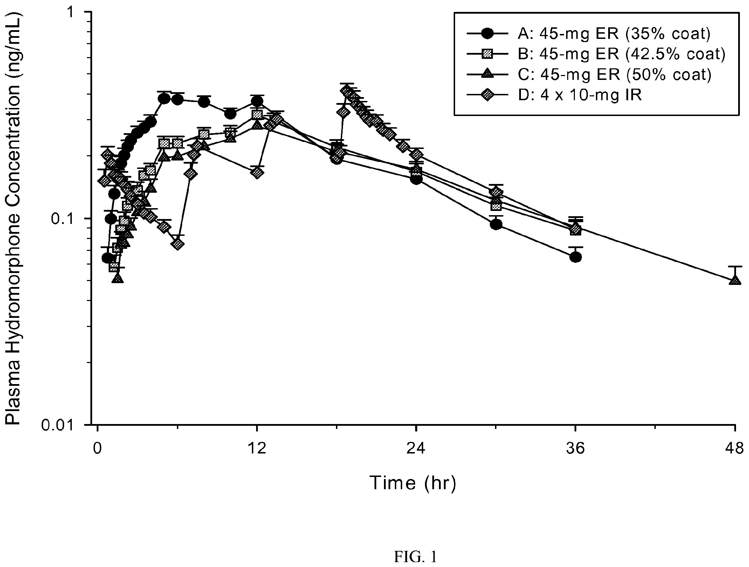

Results of the study are shown in FIG. 1 and Table 3, below. **Table 3.**

| Mean (SD) Pharmacokinetic Parameters for Hydrocodone in Healthy Volunteers Administered Single Doses of 45 mg Hydrocodone ER or 4 x 10 mg Hydrocodone IR | | | | |
|---|---|---|---|---|
| **Dosage** | **Cmax(ng/mL) Mean ± Std Dev** | **Tmax (hr) Median (range)** | **AUC0-inf (ng·h/mL) Mean ± Std Dev** | **T1/2 (hr) Mean ± Std Dev** |
| **45-mg ER 35% Coat** | 49.2 ± 13.6 | 5.9 (5.0 - 8.0) | 640 ± 187 | 11.7 ± 4.5 |
| **45-mg ER 42.5% Coat** | 32.6 ± 7.7 | 8.0 (5.0 - 11.9) | 600 ± 165 | 11.4 ± 3.4 |
| **45-mg ER 50% Coat** | 28.4 ± 7.5 | 8.0 (5.0 - 11.9) | 578 ± 188 | 11.3 ± 4.0 |
| **4 X 10-mg IR** | 37.3 ± 8.8 | 1.0 (0.5-4.0) | 581 ± 167 | 9.1 ± 4.0 |

### Example 4 Effects of food and alcohol on PK parameters

This was a Phase 1, single-center, randomized, open-label, 5-period crossover study to characterize the pharmacokinetics of hydrocodone bitartrate following administration of a 15 mg hydrocodone bitartrate extended-release tablets made according to Lot 200923, shown in Table 2 above (coated granules with 42.5% coating level) with water in a fasted state, with water in a fed state, and with varying amounts of alcohol (4, 20 and 40% v/v) in a fasted state.

Subjects were randomly assigned to 1 of the following 5 treatment sequences: ABCDE, BCDEA, CDEAB, DEABC, or EABCD. The treatments are described in Table 4, below.

**Table 4.**

| Fed and Fasted Treatments | |
|---|---|
| **Treatment** | **How administered** |
| A | With 240 mL of water in fasted state |
| B | With 240 mL of water in fed state |
| C | With 240 mL of 4% (v/v) ethanol in a fasted state |
| D | With 240 mL of 20% (v/v) ethanol in a fasted state |
| E | With 240 mL of 40% (v/v) ethanol in a fasted state |

All subjects began fasting at approximately 2200 on the evening before study drug administration. Subjects receiving treatments A, C, D, and E remained fasting for 4 hours after administration in each administration period. Subjects receiving treatment B fasted until approximately 30 minutes prior to study drug administration at which time they were provided a standard high-fat breakfast and then remained fasting until a minimum of 4 hours after study drug administration.

There was a minimum 5-day washout between successive administrations of study drug. Subjects received each of the 5 treatments once. Subjects received one 50-mg tablet of naltrexone hydrochloride with 240 mL of water to block opioid receptors and minimize opioid-related adverse events approximately 15 hours and 3 hours before each study drug administration and approximately 9 hours and 21 hours after each study drug administration.

For each of the 5 treatment periods, venous blood samples for pharmacokinetic analyses were collected immediately (within approximately 5 minutes) before each study drug administration and 15, 30, and 45 minutes, and 1, 1.25, 1.5, 1.75, 2, 2.25, 2.5, 3, 3.5, 4, 5, 6, 8, 10, 12, 18 24, 30, 36, 48, 60, and 72 hours after each study drug administration. Concentrations of hydrocodone and hydromorphone were determined in human plasma samples using a validated high-performance liquid chromatography method with tandem mass spectrometric detection (LC-MS/MS).

**Table 5.**

| Mean (+/- SD) Pharmacokinetic Parameters for Hydrocodone in Healthy Volunteers Administered Single Doses of 15 mg Hydrocodone ER Tablets under Fasted or Fed Conditions or with Ethanol. FIG. 2 provides a graph of the data in this Table. | | | | |
|---|---|---|---|---|
| **Dosage** | **Cmax(ng/mL) Mean ± Std Dev** | **Tmax (hr) Median (range)** | **AUC0-inf (ng·h/mL) Mean ± Std Dev** | **T1/2 (hr) Mean ± Std Dev** |
| 15-mg ER Fasted | 12.8 ± 3.2 | 8.0 (5.0-10.0) | 198.2 ± 53.8 | 10.8 ± 5.3 |
| 15-mg ER Fed | 19.0 ± 4.7 | 6.0(3.0-10.0) | 216.7 ± 51.4 | 8.6 ± 3.6 |
| 15-mg ER 4% Ethanol | 13.6 ± 3.6 | 8.0 (5.0- 12.0) | 214.3 ± 53.2 | 9.9 ± 3.9 |
| 15-mg ER 20% Ethanol | 14.0 ± 3.9 | 8.0 (4.0-10.0) | 228.2 ± 63.5 | 10.5 ± 3.9 |
| 15-mg ER 40% Ethanol | 13.6 ± 2.9 | 6.0 (3.5-12.0) | 219.7 ± 58.7 | 11.8 ± 4.9 |

As shown in Table 5 above, the tested dosage form was resistant to food effect (only a 25% change in Tmax and 48% change in Cmax), and was resistant to ethanol dose dumping (for example, 6% change in Cmax comparing the 40% ethanol samples to the fasted no ethanol samples).

### Comparative Example 5 - Effects of food on Formulation w/o Viscosity Modifier

Using a process similar to that described in Examples 1 and 14 from publication US2008/0069891, granules were formed having the following formulation:

**Table 6.**

| Granule formulation | |
|---|---|
| **Ingredient** | **Amount (% w/w)** |
| Oxycodone HCl | 46.1 |
| Hydroxypropyl methylcellulose (HPMC) | 36.9 |
| Ethylcellulose | 17.0 |
| Total | 100.00 |

**Table 7.**

| Coating formulation | |
|---|---|
| **Ingredient** | **Amount (% w/w)** |
| Oxycodone granules (oxycodone HCl, | 52.5 |
| HPMC, ethylcellulose) | |
| Ethylcellulose | 31.7 |
| Magnesium stearate | 15.8 |
| **Total** | 100.00 |

The granules were then combined with the matrix materials provided in Table 8 and compressed into tablets.

**Table 8.**

| Matrix formulation | | |
|---|---|---|
| Component | Amount (% w/w) | Amount (mg) |
| **Oxycodone coated granules** | **38.89*** | **330.6** |
| Lactose Monohydrate (fast Flo) | 51.11 | 434.4 |
| COMPRITOL (glyceryl behenate) | 10.00 | 85.0 |
| **Total** | **100.00** | **850.0 mg** |

| | | |
|---|---|---|
| *This percentage assume coated granules potency of 100% | | |

While COMPRITOL was always kept at 10 % of the total weight of the dosage form (tablet), any change in the actual assay amount, from theoretical values, is accounted for by changing the amount of lactose and coated granules to maintain the amount of Oxycodone HCl at 80 mg per tablet. The average tablet weight is 850 mg, and has an average hardness of between 140 and 155 N. The tablet dimensions are .3125" x .5625".

The above described tablets were then used in a Phase 1, single-center, randomized, open-label, 3-period study to assess the effect of food on the single-dose pharmacokinetics of 80-mg oxycodone hydrochloride extended release tablets and to characterize the single- and multiple-dose pharmacokinetics of 80-mg oxycodone hydrochloride extended release tablets in healthy subjects.

Subjects were randomly assigned to 1 of 2 treatment sequences: ABC or BAC, whereby A was a single dose of the 80-mg oxycodone hydrochloride extended release tablet administered with the subject in a fasted state, B was a single dose of the 80-mg oxycodone hydrochloride extended release tablet administered with the subject in a fed state, and C was one 80-mg oxycodone hydrochloride extended release administered twice daily (bid) for 4.5 days (data from treatment group C not shown).

The study consisted of a screening visit (visit 1) within 21 days before the 1st dose of study drug, followed by 2 open-label single-dose administration periods (periods 1 and 2, visits 2 and 3); 1 open-label, 4.5-day, multiple-dose administration period (period 3, included in visit 3); and a follow-up visit (visit 4). There was a minimum 5-day washout between administration of study drug in periods 1 and 2. Administration period 3 began immediately after collection of the 48-hour pharmacokinetic sample in administration period 2.

Subjects received all 3 treatments during the study. Subjects received 50 mg of naltrexone with 240 mL of water to block opioid receptors and minimize opioid-related adverse events approximately 15 and 3 hours before administration and approximately 9 and 21 hours after administration in periods 1 and 2. Additionally, during administration period 2, subjects received naltrexone approximately 33 and 45 hours after study drug administration (in preparation for study drug administration in period 3).

During administration period 3, subjects received naltrexone every 12 hours through 21 hours after the last study drug administration on day 5.

Subjects were required to fast (no food or beverages) overnight beginning at approximately 2100 hours on the evening prior to study drug administration in periods 1 and 2. Subjects randomly assigned to Treatment A continued to fast for a minimum of 4 hours after study drug administration. Subjects randomly assigned to Treatment B fasted until approximately 30 minutes prior to study drug administration, at which time they were provided a standard high-fat breakfast, which must have been consumed in its entirety prior to dosing. Subjects receiving Treatment B were then required to remain fasting until a minimum of 4 hours after study drug administration. All subjects (irrespective of randomized treatment) were permitted to have nonmineral water up to 1 hour before and starting 1 hour after each study drug administration.

During the administration period for Treatments A and B, blood samples (3 mL) were collected by venipuncture or indwelling catheter. Samples were collected immediately (within approximately 5 minutes) before each study drug administration and 15, 30, and 45 minutes and 1, 1.5, 2, 2.5, 3, 3.5, 4, 5, 6, 7, 8, 10, 12, 16, 24, 36, and 48 hours after each study drug administration.

In this study, 30 subjects were enrolled and randomly assigned to a treatment sequence; all 30 subjects received at least 1 dose of study drug; 25 (83%) subjects were evaluable for pharmacokinetic analysis; and 23 (77%) subjects completed the study.

**Table 9.**

| | **Mean (+/- SD) Pharmacokinetic Parameters for Oxycodone in Healthy Volunteers of 80-mg Oxycodone ER Tablets under Fasted or Fed Conditions** | |
|---|---|---|
| **Parameter** | **Oxycodone ER (Fasted)** | **Oxycodone ER (Fed)** |
| Cmax (ng/mL) | 81.9 ± 22.23 | 135.1 ± 20.47 |
| tmax (hr)a | 8.0 (3.0-12.0) | 5.0 (4.0-10.0) |
| AUC0-∞ (ng·hr/mL) | 1145.8±234.70 | 1218.8±253.97 |

As seen above in Table 9, the samples from patients given the tested formulation showed that the formulation was not very resistant to food effect (e.g. a percent change of 65% between fasted and fed states).

## Claims

1. A sustained-release oral dosage form comprising:
a matrix, wherein the matrix comprises a first viscosity modifier in an amount from 1 to 10 percent by weight of the dosage form, and
coated granules comprising:
a granule comprising hydrocodone or a salt form thereof in an amount from 0.1 to 90 percent by weight of the granule, a first strong film former in an amount from 1 to 90 percent by weight of the granule, a second viscosity modifier in an amount from 1 to 90 percent by weight of the granule, wherein the granule does not contain a fat/wax,
a coating on the granule, wherein the coating is present in an amount from 20 to 80 percent by weight of the coated granule, wherein the coating comprises a second strong film former in an amount from 10 to 50 percent by weight of the coated granule, and a fat/wax in an amount from 10 to 25 percent by weight of the coated granule,
wherein first and second strong film formers are independently selected from the group consisting of: ethylcellulose; Ammonio Methacrylate Copolymer, Type B; Ammonio Methacrylate Copolymer, Type A; Amino Methacrylate Copolymer; Ethyl Acrylate and Methyl Methacrylate Copolymer Dispersion; Methacrylic Acid Copolymer, Type A; Methacrylic Acid Copolymer, Type B; and shellac, and
wherein the first and second viscosity modifiers are independently selected from the group consisting of: sodium alginate, hydroxypropylmethylcellulose, hydroxyethylcellulose, hydroxypropylcellulose, methylcellulose, carboxymethylcellulose, sodium carboxymethylcellulose, polyvinylpyrrolidone, crosslinked polyacrylic acid, gelatin, pectins, gums, polyethylene oxides, Konjac flour, carrageenan, xanthan gum, or mixtures thereof

2. The dosage form of claim 1, wherein the hydrocodone is hydrocodone bitartrate.

3. The dosage form of claim 1, wherein the first and/or second viscosity modifier is a gelling polymer.

4. The dosage form of claim 3, wherein the gelling polymer is selected from the group consisting of: hydroxypropylmethylcellulose, hydroxypropylcellulose, methylcellulose, hydroxyethylcellulose, and carboxymethylcellulose.

5. The dosage form of claim 4, wherein the gelling polymer is hydroxypropylmethylcellulose.

6. The dosage form of claim 1, wherein the first viscosity modifier is present in an amount from 5 to 10 percent by weight of the dosage form.

7. The dosage form of claim 1, wherein the % change in Cmax between the fasted versus fed state is less than 50%.

8. The dosage form of claim 1, wherein the % change in Tmax between the fasted versus fed state is less than 35%.

9. The dosage form of claim 1, wherein the dosage form is crush resistant.

10. The dosage form of claim 1, wherein the dosage form is resistant to alcohol dose dumping.

11. The dosage form of claim 1, wherein the first strong film former and the second strong film former are the same.

12. The dosage form of claim 1, wherein the fat/wax is selected from the group consisting of glycerol fatty esters, fatty glyceride derivatives, waxes, fatty alcohols.

## Patentansprüche

1. Orale Retardarzneiform, umfassend:
eine Matrix, wobei die Matrix ein erstes Mittel zur Veränderung der Viskosität in einer Menge von 1 bis 10 % des Gewichts der Arzneiform umfasst, und beschichtete Körnchen, umfassend:
ein Körnchen, umfassend Hydrocodon oder ein Salz davon in einer Menge von 0,1 bis 90 % des Gewichts des Körnchens, einen ersten starken Filmbildner in einer Menge von 1 bis 90 % des Gewichts des Körnchens, ein zweites Mittel zur Veränderung der Viskosität in einer Menge von 1 bis 90 % des Gewichts des Körnchens, wobei das Körnchen kein Fett/Wachs enthält,
eine Beschichtung auf dem Körnchen, wobei die Beschichtung in einer Menge von 20 bis 80 % des Gewichts des beschichteten Körnchens vorliegt, wobei die Beschichtung einen zweiten starken Filmbildner in einer Menge von 10 bis 50 % des Gewichts des beschichteten Körnchens umfasst, und ein Fett/Wachs in einer Menge von 10 bis 25 % des Gewichts des beschichteten Körnchens,
wobei die ersten und zweiten starken Filmbildner unabhängig ausgewählt sind aus der Gruppe bestehend aus: Ethylcellulose; Ammoniummethacrylat-Copolymer, Typ B; Ammoniummethacrylat-Copolymer, Typ A; Aminomethacrylat-Copolymer; Ethylacrylat und Methylmethacrylat-Copolymerdispersion;
Methacrylsäure-Copolymer, Typ A; Methacrylsäure-Copolymer, Typ B; und Schellack, und wobei das erste und zweite Mittel zur Veränderung der Viskosität unabhängig ausgewählt sind aus der Gruppe bestehend aus: Natriumalginat, Hydroxypropylmethylcellulose, Hydroxyethylcellulose, Hydroxypropylcellulose, Methylcellulose, Carboxymethylcellulose, Natriumcarboxymethylcellulose, Polyvinylpyrrolidon, quervernetzter Polyacrylsäure, Gelatine, Pektinen, Gummis, Polyethylenoxiden, Konjakmehl, Carrageenan, Xanthan, oder Gemischen davon.

2. Arzneiform nach Anspruch 1, wobei das Hydrocodon Hydrocodonbitartrat ist.

3. Arzneiform nach Anspruch 1, wobei das erste und/oder zweite Mittel zur Veränderung der Viskosität ein gelierendes Polymer ist.

4. Arzneiform nach Anspruch 3, wobei das gelierende Polymer ausgewählt ist aus der Gruppe bestehend aus: Hydroxypropylmethylcellulose, Hydroxypropylcellulose, Methylcellulose, Hydroxyethylcellulose, und Carboxymethylcellulose.

5. Arzneiform nach Anspruch 4, wobei das gelierende Polymer Hydroxypropylmethylcellulose ist.

6. Arzneiform nach Anspruch 1, wobei das erste Mittel zur Veränderung der Viskosität in einer Menge von 5 bis 10 % des Gewichts der Arzneiform vorliegt.

7. Arzneiform nach Anspruch 1, wobei die prozentuale Änderung in Cmax zwischen dem gefasteten und dem gespeisten Zustand weniger als 50 % beträgt.

8. Arzneiform nach Anspruch 1, wobei die prozentuale Änderung in Tmax zwischen dem gefasteten und dem gespeisten Zustand weniger als 35 % beträgt.

9. Arzneiform nach Anspruch 1, wobei die Arzneiform druckfest ist.

10. Arzneiform nach Anspruch 1, wobei die Arzneiform resistent gegenüber Alkohol-Sturzentleerung ist.

11. Arzneiform nach Anspruch 1, wobei der erste starke Filmbildner und der zweite starke Filmbildner derselbe sind.

12. Arzneiform nach Anspruch 1, wobei das Fett/Wachs ausgewählt ist aus der Gruppe bestehend aus Glycerinfettsäureestern, Fettglyceridderivaten, Wachsen, Fettalkoholen.

## Revendications

1. Forme galénique orale à libération prolongée comprenant :
une matrice, où la matrice comprend un premier modificateur de viscosité à une teneur comprise entre 1 et 10 pour cent en masse de la forme galénique, ainsi que des granules enrobés comprenant :
un granule comprenant de l'hydrocodone ou une forme saline de celle-ci à une teneur comprise entre 0,1 et 90 % en masse du granule, un premier agent filmogène fort à une teneur comprise entre 1 et 90 % en masse du granule, un deuxième modificateur de viscosité à une teneur comprise entre 1 et 90 % en masse du granule, où le granule ne contient pas de matière grasse/cire, un enrobage sur le granule, où l'enrobage est présent à une teneur comprise entre 20 et 80 % en masse du granule enrobé, où l'enrobage comprend un deuxième agent filmogène fort à une teneur comprise entre 10 et 50 % en masse du granule enrobé, et une matière grasse/cire à une teneur comprise entre 10 et 25 % en masse du granule enrobé,
où les premier et deuxième agents filmogènes forts sont indépendamment choisis dans le groupe constitué par les suivants : éthylcellulose ; copolymère d'ammoniométhacrylate, Type B ; copolymère d'ammoniométhacrylate, Type A ; copolymère d'aminométhacrylate ; dispersion de copolymère d'acrylate d'éthyle et de méthacrylate de méthyle ; copolymère d'acide méthacrylique, Type A ; copolymère d'acide méthacrylique, Type B ; et gomme laque, et
où les premier et deuxième modificateurs de viscosité sont indépendamment choisis dans le groupe constitué par les suivants : alginate de sodium, hydroxypropylméthylcellulose, hydroxyéthylcellulose, hydroxypropylcellulose, méthylcellulose, carboxyméthylcellulose, carboxyméthylcellulose de sodium, polyvinylpyrrolidone, acide polyacrylique réticulé, gélatine, pectines, gommes, oxydes de polyéthylène, farine de Konjac, carraghénane, gomme xanthane, ou leurs mélanges.

2. Forme galénique selon la revendication 1, où l'hydrocodone est le bitartrate d'hydrocodone.

3. Forme galénique selon la revendication 1, où le premier et/ou deuxième modificateur de viscosité est un polymère gélifiant.

4. Forme galénique selon la revendication 3, où le polymère gélifiant est choisi dans le groupe constitué par les suivants : hydroxypropylméthylcellulose, hydroxypropylcellulose, méthylcellulose, hydroxyéthylcellulose et carboxyméthylcellulose.

5. Forme galénique selon la revendication 4, où le polymère gélifiant est l'hydroxypropylméthylcellulose.

6. Forme galénique selon la revendication 1, où le premier modificateur de viscosité est présent à une teneur comprise entre 5 et 10 % en masse de la forme galénique.

7. Forme galénique selon la revendication 1, où le pourcentage de variation de Cmax entre l'état à jeun et l'état après repas est inférieur à 50 %.

8. Forme galénique selon la revendication 1, où le pourcentage de variation de Tmax entre l'état à jeun et l'état après repas est inférieur à 35 %.

9. Forme galénique selon la revendication 1, où la forme galénique est résistante à l'écrasement.

10. Forme galénique selon la revendication 1, où la forme galénique est résistante à la dilution de la dose dans l'alcool.

11. Forme galénique selon la revendication 1, où le premier agent filmogène fort et le deuxième agent filmogène fort sont identiques.

12. Forme galénique selon la revendication 1, où la matière grasse/cire est choisie dans le groupe constitué par les esters gras de glycérol, les dérivés de glycéride gras, les cires et les alcools gras.
